# FASCICULE DE BREVET EUROPEEN

(11) **EP 0 516 532 B1**
(45) Date de publication et mention de la délivrance du brevet: **15.01.1997**
(21) Numéro de dépôt: 92401443.4
(22) Date de dépôt: 26.05.1992
(51) Int. Cl.: C07D 311/20, C07D 491/16, C07C 69/716, C12Q 1/37

(54) **Dérivés de coumarines hydrosolubles, leur préparation et leur utilisation comme substrat d'enzyme ou pour la préparation de tels substrats**
Wasserlösliche Cumarderivate, ihre Herstellung und ihre Verwendung als Enzymsubstrate oder für die Zubereitung von solchen Substraten
Watersoluble coumarin derivatives, their preparation and their use as enzyme substrates or for the preparation of those substrates

(30) Priorité: 30.05.1991 FR 9106551
(43) Date de publication de la demande: 02.12.1992
(73) Titulaire: LABORATOIRES EUROBIO, 91953 Les Ulis Cédex B (FR)
(72) Inventeur: Chalom, Joseph, F-75012 Paris (FR); Griffoul, Christine, F-93110 Rosny Sous Bois (FR); Tod, Michel, F-95580 Margency (FR); Reveilleau, Stéphane, F-75006 Paris (FR)
(74) Mandataire: Des Termes, Monique

(56) Documents cités:
- EP-A- 0 067 409
- EP-A- 0 076 379
- WO-A-80/02295
- US-A- 3 784 600
- PATENT ABSTRACTS OF JAPAN, vol. 9, no. 188 (C-295)(1911), 03 août 1985
- CHEMICAL & PHARMACEUTICAL BULLETIN, vol. 25, no. 2, février 1977, Tokyo (JP); Y. KANAOKA et al., pp. 362-363
- CHEMICAL & PHARMACEUTICAL BULLETIN, vol. 36, no. 9, septembre 1988, Tokyo (JP); E. SATO et al., pp. 3496-3502
- CHEMICAL ABSTRACTS, vol. 112, no. 16, 16 avril 1990, Columbus, OH (US); M. TOD et al., p. 825, no. 151038a

## Description

La présente invention a pour objet de nouveaux dérivés de coumarine hydrosolubles et leur procédé de préparation à partir de nouveaux esters β-cétoniques à groupement hydrophile.

Les coumarines sont une importante famille de molécules présentant des applications très variées.

En effet, en dehors de domaines comme la pharmacologie où elles peuvent jouer le rôle d'anticoagulant, les coumarines sont très utilisées pour leurs propriétés optiques (fluorescence et chimiluminescence), par exemple en tant que
- colorants pour lasers,
- produits fluorescents pour l'optique,
- produits pour la synthèse de substrats d'enzymes utilisés dans des dosages pour le diagnostic médical, et
- marqueurs de molécules biologiques, utiles également dans le domaine du diagnostic médical.

Or, pour ces diverses applications, les coumarines ont l'inconvénient d'avoir une solubilité dans l'eau ou, dans des solvants hydrophiles comme les alcools et même dans des solvants mixtes (mélange eau-solvant organique) médiocre ou mauvaise, ce qui diminue leurs performances et leurs possibilités d'emploi.

Dans le cas de la préparation d'un substrat d'enzyme, on réalise le plus souvent une combinaison chimique entre a) un colorant ou un produit fluorescent et b) une molécule biochimique telle qu'un acide aminé, un peptide, un sucre ou une molécule d'acide phosphorique. Le substrat ainsi formé doit présenter les propriétés suivantes :
1°) les caractéristiques de coloration ou de fluorescence de a) doivent être annulées ou très fortement diminuées dans le substrat,
2°) le substrat doit être capable de réagir avec des enzymes spécifiques pour subir un clivage,
3°) le clivage doit régénérer le produit colorant ou fluorescent a) et restaurer ainsi la couleur ou la fluorescence, et
4°) cette restauration doit être proportionnelle à la quantité d'enzyme mise en oeuvre pour que les substrats puissent servir au dosage d'enzyme.

Parmi les dérivés de coumarine connus, on a utilisé jusqu'à présent, pour la préparation de substrats d'enzymes, la 7-amino-4-méthyl coumarine, l'acide 7-amino-coumarine-4-méthane sulfonique et la 7-amino-4-trifluorométhyl coumarine, comme il est décrit dans Chem. Pharm. Bull, vol. 25, 1977, p. 362-363, dans Chem. Pharm. Bull., vol. 36, 1988, p. 3496-3502 et dans WO 80/02295.

Couplée à la leucine, la 7-amino-4-méthyl coumarine peut être utilisée par exemple comme substrat pour la L-leucine amino peptidase. Cette coumarine est très intéressante car elle est très fluorescente avec un maximum d'intensité de fluorescence, environ 14 fois plus élevé que celui des amines utilisées habituellement comme substrat, telles que la β-naphtylamine. Par ailleurs, il y a une différence d'intensité de fluorescence importante entre l'aminocoumarine et le substrat correspondant amide.

Cependant, comme il est indiqué dans Chem. Pharm. Bull. vol. 36, p. 3496-3502 (1988), on doit utiliser avec cette aminocoumarine un mélange d'eau et de solvant organique tel que le sulfoxyde de diméthyle en raison de sa solubilité trop faible dans l'eau, ce qui constitue un inconvénient car de nombreuses enzymes comme l'encéphalinase sont sensibles aux solvants organiques ; de plus la présence de solvant organique complique la procédure de dosage.

Aussi, pour améliorer la solubilité dans l'eau de cette coumarine, on a remplacé le groupement méthyle en position 4 par le groupement méthane sulfonique plus hydrophile.

Cependant, la synthèse de cette dernière aminocoumarine est assez compliquée ; de plus, elle ne convient pas pour de nombreuses enzymes.

La présente invention a précisément pour objet de nouveaux dérivés de coumarines hydrosolubles qui pallient ces inconvénients, grâce à la présence en position 4 du noyau coumarine d'une chaîne polyéther leur conférant une solubilité dans l'eau améliorée, sans modifier pour autant leurs propriétés fluorescentes ou colorantes.

Ces dérivés de coumarine répondent à la formule : 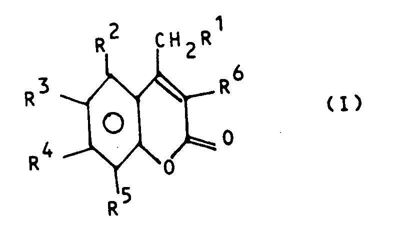 dans laquelle
1) R¹ représente un radical répondant aux formules
   - (OCH₂CH₂)ₘOCH₂R⁷, ou
   - (OCH(CH₃)CH₂)ₘOCH₂R₇,
   dans lesquelles m est un nombre entier de 1 à 30, et R⁷ représente
   - un atome d'hydrogène,
   - un atome d'halogène,
   - OH,
   - OR⁸,
   - SH,
   - SR⁸,
   - COOH,
   - COOR⁹,
   - CONHR⁸, CONR⁸R¹⁰, CONHNH₂, 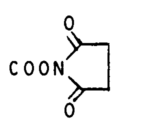
   - NH₂,
   - NHR⁸,
   - NR⁸R¹⁰,
   - SO₃H,
   - SO₃R⁹, ou
   - un radical alkyle, alcoxy ou aryle, non substitué ou substitué par au moins un substituant choisi parmi les atomes d'halogène, le radical phényle, CF₃, OH, OR⁸, SH, SR⁸, COOH, COOR⁹, NH₂, NHR⁸, NR⁸R¹⁰ SO₃H et SO₃R⁹,
      où R⁸ est un radical alkyle ou aryle, R⁹ est un radical alkyle ou aryle, NH₄ ou M_{1/v} avec M étant un métal de valence v, et R¹⁰ est un radical alkyle ou aryle, ou R⁸ et R¹⁰ peuvent former ensemble un cycle hydrocarboné, saturé ou insaturé comportant éventuellement un hétéroatome choisi parmi O, S et N,
2) R², R³, R⁴ et R⁵ qui peuvent être identiques ou différents, représentent
   - un atome d'hydrogène,
   - un atome d'halogène,
   - NH₂,
   - NHR⁸,
   - NR⁸R¹⁰,
   - NHR¹¹,
   - NR⁸R¹¹,
   - SH
   - SR⁸
   - SR¹¹
   - OH
   - OR⁸
   - OR¹¹
      avec R⁸ et R¹⁰ ayant la signification donnée ci-dessus, et R¹¹ représentant un radical acyle dérivé d'un composé choisi parmi les acides aminés protégés ou non protégés, les peptides protégés ou non protégés, les acides carboxyliques et les acides gras, le radical PO₃R₂ avec R étant un atome d'hydrogène ou un radical alkyle, ou un radical dérivé d'un sucre, ou
   - un radical alkyle ou alcoxy, non substitué ou substitué par au moins un substituant choisi parmi les atomes d'halogène, OH, OR⁸, SH, SR⁸, COOH, COOR⁹, NH₂, NHR⁸, NR⁸R¹⁰, SO₃H, SO₃R⁹, SR¹¹, OR¹¹, CF₃, NHR¹¹ et NR⁸R¹¹ avec R⁸, R⁹, R¹⁰ et R¹¹ ayant la signification donnée ci-dessus, ou
3) R² et R³, R³ et R⁴ ou R⁴ et R⁵ forment ensemble un noyau cyclique saturé ou insaturé de 5 à 8 atomes de carbone, ou un noyau hétérocyclique comportant au moins un hétéroatome choisi parmi N, O et S,
   ou
4) R³, R⁴ et R⁵ forment avec le noyau phényle auquel ils sont liés un noyau polycyclique, à trois cycles condensés comportant éventuellement un ou plusieurs hétéroatomes choisis parmi N, O et S, ou
5) R², R³ et R⁴ forment avec le noyau phényle auquel ils sont liés un noyau polycyclique, à trois cycles condensés, comportant éventuellement un ou plusieurs hétéroatomes choisis parmi N, O et S, et
6) R⁶ représente
   - un atome d'hydrogène,
   - un atome d'halogène,
   - COOH,
   - COOR⁹ avec R⁹ ayant la signification donnée ci-dessus, ou
   - un radical alkyle, non substitué ou substitué par au moins un substituant choisi parmi les atomes d'halogène, OH, SH, COOH, COOR⁹, OR⁸, SR⁸, SO₃H et SO₃R⁹ avec R⁸ et R⁹ ayant la signification donnée ci-dessus.

Dans la formule donnée ci-dessus, les termes radical alkyle ou alcoxy désignent des radicaux linéaires ou ramifiés ayant généralement de 1 à 10 atomes de carbone, de préférence 1 à 5 atomes de carbone.

Les termes radical aryle désignent les radicaux dérivés d'hydrocarbures aromatiques comme le benzène, le naphtalène. A titre d'exemple de tels radicaux, on peut citer les radicaux phényle, naphtyle.

Les termes de cycle hydrocarbone désignent des hydrocarbures cycliques saturés ou insaturés ayant de 3 à 10 atomes de carbone.

Lorsqu'ils comportent un ou plusieurs hétéroatomes, il peut s'agir par exemple de la pipéridine, de la pyridine, de la pipérazine, du pyrrole, du furanne, du pyranne, de la morpholine.

Les acides aminés utilisés pour R¹¹ peuvent être les acides aminés naturels, en particulier ceux servant à faire des substrats pour des enzymes.

Ces acides peuvent être protégés au niveau de la fonction amine libre (par exemple par des groupements carbobenzoxy, fluorénylméthoxycarbonyle, t-butoxy-carbonyle). On inclut aussi un dérivé d'acide aminé qui est l'acide pyroglutamique.

Les peptides utilisés sont ceux formés de plusieurs acides aminés, en particulier les acides aminés naturels. Dans ces peptides, des acides aminés dextrogyres ou synthétiques peuvent être intégrés dans la chaîne. Les peptides peuvent par ailleurs être modifiés au niveau de la fonction amine restée libre (par exemple par des groupements carbobenzoxy, fluorénylméthoxycarbonyle, t-butoxy carbonyle, succinyle).

Les sucres utilisés pour R¹¹ peuvent être des oses tels que le glucose, le mannose, le galactose.

Si R¹¹ est un radical carbobenzoxyarginyle, on obtient un substrat de la trypsine ; si R¹¹ est un radical pyroglutamyle, on obtient un substrat de la pyroglutamyl amino peptidase ; si R¹¹ est un radical γ-glutamyle, on a un substrat de la γ-glutamyltransférase.

Lorsque R² et R³, R³ et R⁴ ou R⁴ et R⁵ forment un cycle, il peut s'agir des cycles hydrocarbonés et des hétérocycles mentionnés ci-dessus.

Lorsque R², R³ et R⁴ ou R³, R⁴ et R⁵ forment avec le noyau phényle, un noyau polycyclique comportant éventuellement un ou plusieurs hétéroatomes, il peut s'agir par exemple du noyau suivant :

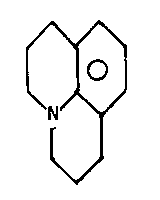

Les métaux M représentant R⁹ peuvent être par exemple des métaux alcalins comme le sodium.

Dans la formule (I) ci-dessus, la présence du radical R¹ qui comporte une chaîne polyéther, permet de conférer aux dérivés de coumarine les propriétés d'hydrosolubilité, tandis que la présence d'au moins un substituant R², R³, R⁴ ou R⁵ permet de conférer aux dérivés, d'autres propriétés, par exemple, renforcer les propriétés fluorescentes et chimiluminescentes de la coumarine, constituer un substrat d'enzyme, ou permettre la synthèse de substrats d'enzymes utilisables pour des dosages d'enzymes.

Aussi, selon un premier mode de réalisation de l'invention, au moins l'un des R², R³, R⁴ et R⁵ représente NH₂, NHR⁸, NHR¹¹, NR⁸R¹¹, SH, OH, SR¹¹ ou OR¹¹ avec R⁸ et R¹¹ ayant la signification donnée ci-dessus.

De préférence, dans ce premier mode de réalisation de l'invention, un seul des R², R³, R⁴ et R⁵ représente NH₂, NHR⁸, NHR¹¹, NR⁸R¹¹, SH, OH, SR¹¹ ou OR¹¹. Dans ce cas, les autres représentent généralement un atome d'hydrogène.

De préférence encore, R², R³ et R⁵ représentent un atome d'hydrogène et R⁴ représente l'un des substituants précités.

Ce premier mode de réalisation est particulièrement adapté à l'utilisation des dérivés de coumarine comme substrats d'enzymes pour des dosages d'enzyme ou comme intermédiaires pour la préparation de substrats d'enzyme.

En effet, lorsque R², R³, R⁴ ou R⁵ représente un groupe choisi parmi NH₂, NHR⁸, NHR¹¹, SH et OH par exemple NH₂ ou OH, on peut facilement coupler le dérivé de coumarine à un composé approprié pour la réalisation d'un substrat d'enzyme par réaction du composé avec ce groupe substituant. Ces dérivés constituent des produits intermédiaires très intéressants car, à partir d'un seul dérivé, on peut réaliser toute une gamme de substrats d'enzymes en faisant réagir ce produit avec des composés appropriés.

Lorsque R², R³, R⁴ ou R⁵ représente NHR¹¹, NR⁸R¹¹, SR¹¹ ou OR¹¹, R¹¹ est choisi de façon à conférer au dérivé de coumarine la propriété d'être un substrat d'enzyme.

A titre d'exemple de tels substrats, on peut citer le dérivé de coumarine de formule (I) dans lequel R⁴ représente NHR¹¹ avec R¹¹ étant le radical dérivé de la L-leucine de formule :
(CH₃)₂CH-CH₂-C(NH₂)-C(O)-, qui constitue un substrat de la L-leucine aminopeptidase.

A titre d'exemple d'autres substrats, on peut citer les dérivés de coumarine de formule (I) dans laquelle R⁴ représente NHR¹¹ avec R¹¹ étant choisi parmi les radicaux de formule :

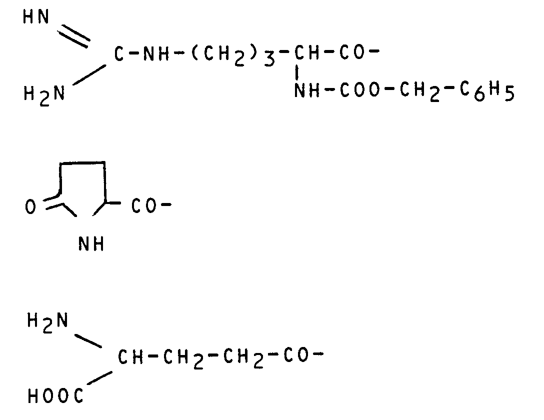

A titre d'exemple de dérivés de coumarine utilisables comme intermédiaires pour la préparation de substrats éventuellement polypeptidiques, on peut citer les dérivés de coumarine de formule (I) dans laquelle R⁴ représente NH₂, OH ou un radical choisi parmi les radicaux de formule :

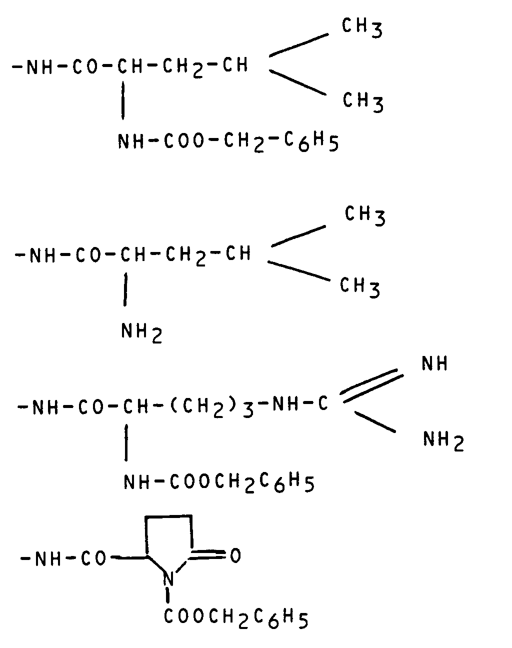

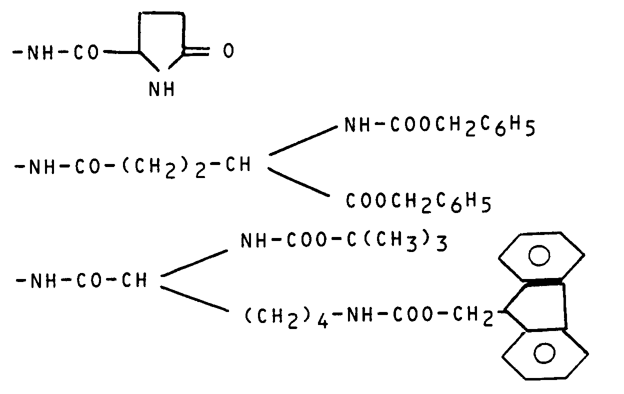

Généralement, dans ce premier mode de réalisation de l'invention R⁶ représente un atome d'hydrogène, mais il pourrait aussi représenter un substituant permettant d'améliorer encore l'hydrosolubilité ou la fluorescence.

Selon un second mode de réalisation de l'invention, plus particulièrement destiné aux dérivés de coumarine utilisés comme produit fluorescent ou comme colorant, au moins l'un des R², R³, R⁴ et R⁵ représente NH₂, NHR⁸, NR⁸R¹⁰, OH, OR⁸, SH, SR⁸ ou un radical alkyle substitué ou non, afin de renforcer les propriétés fluorescentes, ou colorantes, ou l'hydrosolubilités de la coumarine.

Dans une variante de ce second mode de réalisation, R², R³ et R⁴ ou R³, R⁴ et R⁵ forment avec le noyau phényle auquel ils sont liés, un noyau polycyclique comportant trois cycles condensés et un atome d'azote, ce qui permet également de renforcer les propriétés fluorescentes ou colorantes.

Dans les deux modes de réalisation de l'invention, R¹ représente avantageusement -(OCH₂CH₂)ₘOCH₂R⁷.

A titre d'exemple R⁷ peut représenter un atome d'hydrogène, COOR⁹ ou COOH.

Les dérivés de coumarines de l'invention peuvent être préparés par un procédé classique mettant en oeuvre la réaction de Pechman, à partir de nouveaux esters β-cétoniques comportant le groupement hydrophile R¹.

Aussi, l'invention a également pour objet ces nouveaux esters β-cétoniques, qui répondent à la formule :

R¹-CH₂-CO-CH(R⁶)-COOR¹² (II)

dans laquelle R¹ et R⁶ ont la signification donnée ci-dessus, et R¹² représente un radical alkyle, un radical aryle ou un radical cycloalkyle.

De préférence, R⁶ est un atome d'hydrogène.

Les radicaux alkyle ou aryle représentant R¹² peuvent être du même type que ceux décrits précédemment en relation avec la formule (I). Les radicaux cycloalkyle ont généralement de 5 à 7 atomes de carbone.

De préférence, on utilise pour R¹² un radical alkyle ayant de 1 à 4 atomes de carbone, par exemple le radical méthyle ou éthyle.

Ces esters β-cétoniques peuvent être préparés par un procédé classique à partir des acides de formule R¹-CH₂-COOH.

Le procédé de préparation de ces esters β-cétoniques comprend, par exemple les étapes suivantes :
1°) transformer un acide de formule R¹-CH₂-COOH dans laquelle R¹ a la signification donnée ci-dessus, en l'imidazolide ou le chlorure d'acide correspondant,
2°) faire réagir l'imidazolide ou le chlorure d'acide avec un ester de formule : 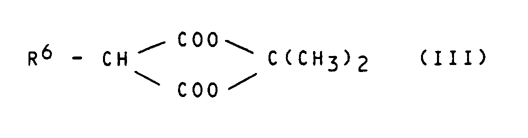 pour former un dérivé acylé, et
3°) faire réagir le dérivé acylé avec un alcool de formule R¹²OH pour obtenir l'ester β-cétonique de formule (II).

Les acides de départ R¹-CH₂-COOH sont des produits du commerce ou peuvent être préparés par des procédés classiques.

L'ester de formule (III) existe dans le commerce (acide de Meldrum) ou peut être préparé par un procédé classique en partant de l'acide de Meldrum ou d'un malonate de diéthyle et en pratiquant deux substitutions successives sur le CH₂ malonique.

Pour préparer les dérivés de coumarines de formule (I) de l'invention, on fait réagir l'ester β-cétonique de formule (II) décrit ci-dessus avec un phénol de formule : 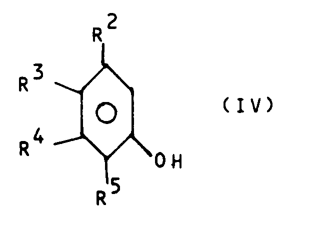 dans laquelle R², R³, R⁴ et R⁵ ont la signification donnée ci-dessus.

Cette réaction de condensation (réaction de Pechmann) peut être effectuée dans l'éthanol en présence d'un catalyseur acide tel que H₂SO₄, HCl, CF₃COOH ou leurs sels, par exemple le chlorure d'aluminium, le chlorure de zinc, le chlorure d'étain.

Lorsque l'un des R², R³, R⁴, R⁵ est un substituant du type NHR¹¹, NR⁸R¹¹, SR¹¹ ou OR¹¹, on préfère généralement préparer tout d'abord le dérivé de coumarine dans lequel ce substituant est NH₂, NHR⁸, SH ou OH et le transformer en le substituant voulu par réaction avec un composé capable d'apporter R¹¹.

Ainsi, lorsque R¹¹ est un radical dérivé d'un acide aminé, on peut faire réagir l'acide aminé (généralement protégé sur sa fonction amine) ou l'acide gras avec le dérivé de coumarine comportant un substituant NH₂ ou NHR⁸, SH ou OH pour former une liaison du type peptide ou du type ester.

Les phénols utilisés pour la réaction peuvent être préparés par des procédés classiques ou sont des produits du commerce.

L'utilisation des esters -cétoniques de formule (II) décrits ci-dessus est intéressante car elle permet d'inclure directement sur le noyau de la coumarine une chaîne du type polyéther sans l'intermédiaire d'un groupe labile, par exemple du type CO-NH, susceptible d'être clivé par certaines enzymes.

Comme on l'a vu plus haut, les dérivés de coumarines correspondant au premier mode de réalisation de l'invention qui comportent un groupement R¹¹ sur l'un des R², R³, R⁴, et R⁵, peuvent être utilisés comme substrats d'enzymes dans un procédé de dosage d'enzymes.

Pour ces dosages, on peut utiliser le dérivé tel quel, ou sous la forme d'un sel d'addition aux acides, par exemple sous la forme de chlorhydrate, bromhydrate, trifluoroacétate.

Pour effectuer un tel dosage, on prépare tout d'abord une solution d'un dérivé de coumarine de formule (I) dans laquelle R², R³, R⁴ ou R⁵ représente NHR¹¹, NR⁸R¹¹, SR¹¹ ou OR¹¹ avec R¹¹ pouvant jouer le rôle de substrat pour l'enzyme à doser, on détermine l'intensité de fluorescence de la solution du dérivé de coumarine ainsi préparé, puis on ajoute à cette solution un échantillon de la solution d'enzyme à doser et on détermine après une durée déterminée, par exemple après 6min, l'intensité de fluorescence de la solution. On détermine ensuite la concentration en enzyme de la solution à doser à partir d'une courbe étalon donnant les variations de l'intensité de fluorescence en fonction de la concentration en enzyme, qui a été élaborée à partir d'échantillons ayant des concentrations connues en enzyme en réalisant les dosages dans les mêmes conditions.

Lors de ce dosage, l'enzyme présente dans l'échantillon à doser hydrolyse le substrat d'enzyme en régénérant le dérivé de coumarine par exemple le dérivé de formule (I) avec R⁴ représentant NH₂, NHR⁸, OH ou SH qui présente une: fluorescence beaucoup plus importante que le dérivé dans lequel R⁴ représente NHR¹¹, SR¹¹, NR⁸R¹¹ ou OR¹¹.

La restauration de l'activité fluorescente est proportionnelle à la quantité d'enzyme de l'échantillon.

D'autres caractéristiques et avantages de l'invention apparaîtront mieux à la lecture des exemples suivants donnés bien entendu à titre illustratif et non limitatif en référence au dessin annexé sur lequel
- Les figures 1, 2, 4, 7, 10, 12, 15, 16, 18, 20, 23, 26, 30, 33, 34, 35 et 37 sont les spectres RMN de composés de l'invention,
- les figures 3, 5, 6, 8, 9, 11, 13, 14, 17, 19, 21, 22, 27, 31, 32, 36 et 38 sont les spectres de masse des composés de l'invention,
- la figure 24 est une courbe représentant la vitesse initiale (en µM/min) de la réaction enzymatique dans le cas d'un substrat enzymatique de l'invention en fonction de la concentration en substrat (en µM),
- la figure 25 est une courbe d'étalonnage représentant l'intensité de fluorescence en fonction de la concentration en enzyme (µg/ml),
- la figure 28 est le spectre d'excitation d'un composé de l'invention, et
- la figure 29 est le spectre d'émission d'un composé de l'invention.

### Exemple 1 : Préparation du 3,6,9-trioxadécanoyle acétate d'éthyle (composé n° 1).

Ce composé est un ester -cétonique de formule (II) avec
- R¹=(OCH₂CH₂)₂OCH₃
- R¹²=C₂H₅.

Dans un premier récipient, on prépare une solution anhydre de 29g d'acide 3,6,9-trioxadécanoïque (0,16mol) dans 400ml de dichlorométhane, puis on introduit dans cette solution, sous agitation, 28,3g (0,174mol) de carbonyldiimidazole et on poursuit l'agitation pendant 2h.

Dans un second récipient, on introduit 24,65g d'acide de Meldrum dans 400ml de dichlorométhane anhydre, puis on refroidit à -5°C et on ajoute 12,9g de pyridine dans 24ml de dichlorométhane.

En maintenant le second récipient à -5°C, on introduit sur une durée de 30min, à l'abri de la lumière, la solution du premier récipient, puis on porte à 40°C pendant 15min.

On ajoute ensuite 165ml d'eau glacée en refroidissant, puis on laisse décanter. On lave alors la phase organique avec 120ml de HCl 2,5N, puis avec 120ml de solution saline saturée, et on évapore jusqu'à siccité.

On reprend alors le résidu dans 500ml d'éthanol anhydre et on porte au reflux pendant 2h30. On évapore sous vide, on distille et on obtient ainsi 12,1g de composé n° 1, ce qui correspond à un rendement de 30%.

Les caractéristiques de ce composé sont les suivantes :
1°) point d'ébullition sous 54Pa : 140-145°C.
2°) Chromatographie sur couche mince (C.C.M.) de silice fluorescente, en utilisant comme éluant un mélange butanol/acide acétique/eau (60:20:20) :
   - Rf = 0,7, révélé sous ultraviolet à 254nm, aux vapeurs d'iode, et à la 2,4 DNPH,
3°) Spectre RNM (résonance magnétique nucléaire)

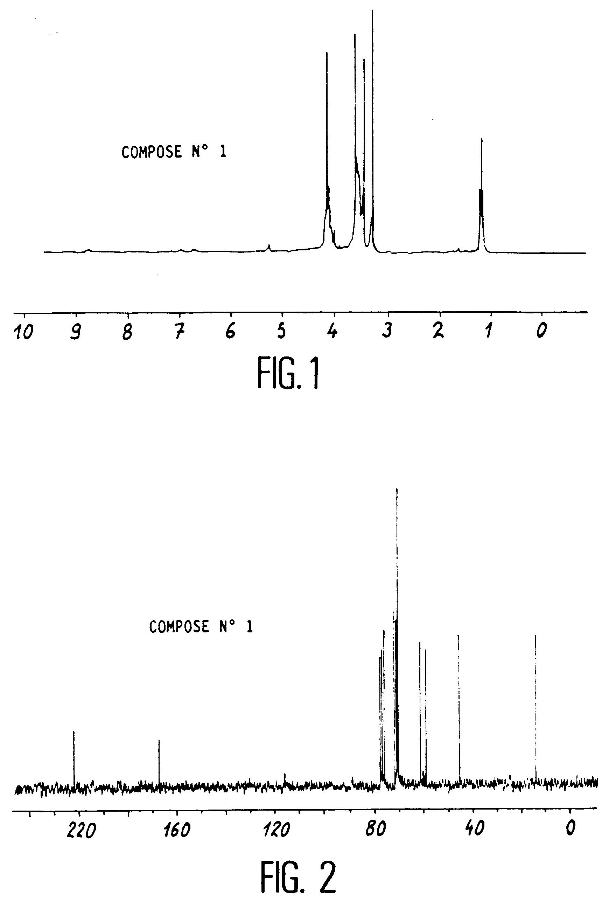

   Les figures 1 et 2 représentent respectivement le spectre (RMN) du proton et du carbone 13.
   Ces spectres confirment bien la structure du produit.

### Exemple 2 : Préparation du 10-carboxybenzyl-3,6,9-décanoyl acétate d'éthyle (composé n°2).

Ce composé est un ester β-cétonique répondant à la formule (II) avec
- R¹=(OCH₂CH₂)₂OCH₂-COO-CH₂-C₆H₅,
- R¹²=éthyle,
- R⁶ = H.

Pour cette préparation, on part de l'acide 3,6,9-trioxaundécane-1,11 dioïque que l'on transforme en ester monobenzylique par couplage en présence de dicyclohexyl carbodiimide. On transforme ensuite cet ester monobenzylique en l'ester β-cétonique en utilisant le même mode opératoire que dans l'exemple 1.

### a) Préparation de l'ester monobenzylique de l'acide 3,6,9-trioxa-undécane-1,11-dioïque. (composé n° 3) de formule :

HOOC-CH₂-(OCH₂CH₂)₂-OCH₂-COO-CH₂-C₆H₅

On introduit 10,8g d'alcool benzylique et 0,7g de aminopyridine (DMAP) sur une solution anhydre de 88,9g d'acide 3,6,9-trioxa-undécane-1,11-dioïque dans 700ml de CH₂Cl₂. En refroidissant à 5°C, on introduit 20,6g de dicyclohexylcarbodiimide (DCCI) dans 40ml de dichlorométhane anhydre. On agite pendant 2h à la température ambiante, puis on filtre le précipité formé. On lave le filtrat avec 150ml d'acide chlorhydrique 1N, 150ml d'eau, puis 2 fois 200ml de bicarbonate de sodium 0,8N. On acidifie la solution de bicarbonate par addition de l'acide chlorhydrique 3N pour ajuster son pH à 2, puis on l'extrait par 3 fois 150ml de dichlorométhane. On évapore les extraits organiques jusqu'à siccité et on obtient ainsi 11,7g d'ester monobenzylique (composé n° 3), ce qui correspond à un rendement de 38%.

Les caractéristiques du produit sont les suivantes :
1°) La chromatographie sur couche mince de silice fluorescente en utilisant comme éluant un mélange de butanol, d'acide acétique et d'eau (60:20:20) donne un Rf de 0,5, révélé sous ultraviolet à 254nm et aux vapeurs d'iode.
2°) Spectrométrie de masse (ionisation à l'ammoniac).

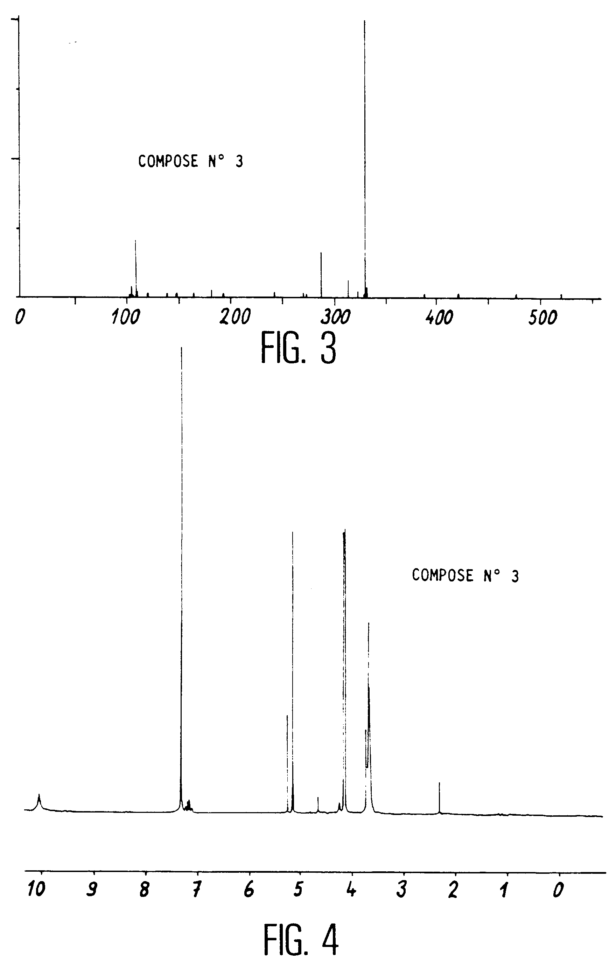

   La figure 3 représente le spectre de masse de ce produit. Le pic à 313 (M+1) et 330 (M+18) confirme la masse du produit qui est de 312.
3°) Spectre RMN (200MHz).
   La figure 4 représente le spectre du proton et confirme la structure du produit.

### b) Préparation du 10-carboxybenzyl-3,6,9-décanoyl acétate d'éthyle (composé n°2).

Dans un premier récipient, on prépare une solution de 10g de l'ester monobenzylique préparé en a) (composé n°3) dans 16ml de dichlorométhane, puis on introduit sous agitation 5,71g (0,035mol) de carbonyl diimidazo.Le et on poursuit l'agitation pendant 2h.

Dans un second récipient, on introduit 5,04g d'acide de Meldrum dans 16ml de dichlorométhane anhydre, puis on refroidit à -5°C et on ajoute 2,5g de pyridine dans 6ml de dichlorométhane.

On introduit ensuite dans ce second récipient, toujours à -5°C, sur une durée de 30min et à l'abri de l'air, la solution d'imidazolide du premier récipient, puis on porte à 40°C pendant 15min.

Sous refroidissement, on ajoute 24ml d'eau glacée, on décante et on lave la phase organique avec 15ml de HCl 2,5N et 15ml de solution saline saturée, puis on évapore la phase organique jusqu'à siccité.

On reprend le résidu dans 100ml d'éthanol anhydre, on porte au reflux pendant 2h30, et on évapore sous vide. On purifie sur une colonne de silice en utilisant comme éluant un mélange CH₂Cl₂/tétrahydrofurane THF (90:10).

On obtient ainsi 4,7g du composé n° 2 (fractions 22 à 43), ce qui correspond à un rendement de 39%.

Les caractéristiques du produit sont les suivantes.
1°) Chromatographie sur couche mince.
   En utilisant la silice fluorescente K₆F et un mélange butanol - acide acétique - eau (60:20:20) comme solvant, on obtient un Rf de 0,55 révélé à 254nm, 365nm, et à l'iode.
2°) Spectrométrie de masse (ionisation chimique à l'ammoniac).

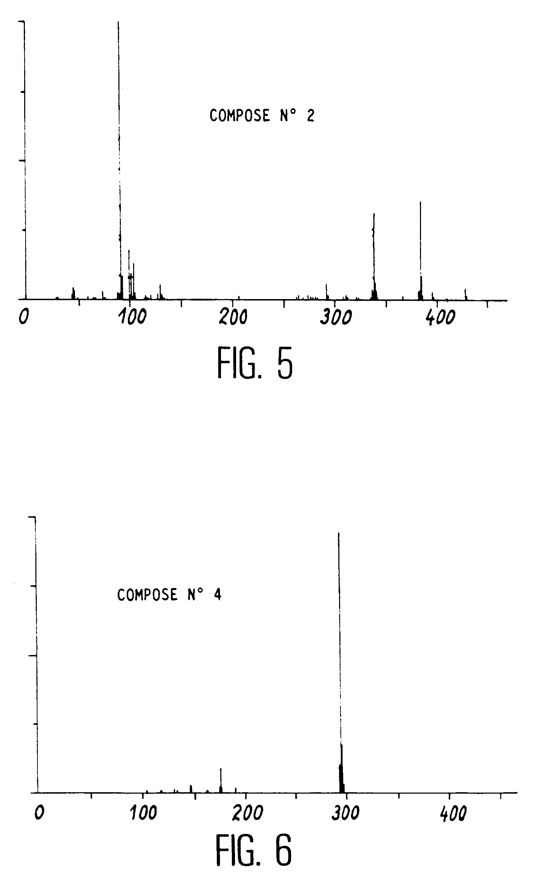

   La figure 5 représente le spectre de masse du composé n° 2.
   Le pic à M+1 = 383 confirme la masse moléculaire du produit (382).

### Exemple 3 : Préparation de la 7-amino-4-(2′,5′,8′-trioxanonyl)coumarine (composé n°4) de formule

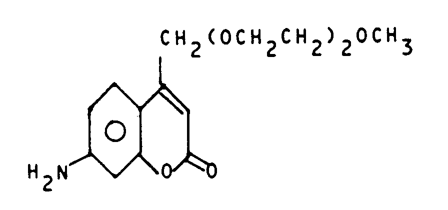

Ce composé répond à la formule (I) avec
- R¹=(OCH₂CH₂)₂OCH₃
- R²=R³=R⁵=R⁶=H, et
- R⁴=NH₂.

Dans un ballon de 100ml, on introduit 15g (0,06mol) de composé n°1, 6,6g (0,06mol) de 3-aminophénol, 45ml d'éthanol absolu et 9,5g de chlorure de zinc. On porte le mélange réactionnel à l'ébullition sous agitation pendant 24h, puis on le verse dans un mélange de 800ml de dichlorométhane et de 600ml d'eau. On laisse décanter, on extrait la phase aqueuse avec deux fois 200ml de dichlorométhane et on lave les phases organiques réunies avec 120ml d'eau. On évapore jusqu'à siccité. On fait cristalliser dans 50ml d'acétate d'éthyle, on purifie le produit brut obtenu sur une colonne de silice en utilisant l'acétate d'éthyle comme éluant et on recristallise dans l'acétate d'éthyle.

On obtient ainsi 2,67g du composé n° 4, ce qui correspond à un rendement de 15%.

Les caractéristiques du produit sont les suivantes.
1°) CCM (silice fluorescente K₆F, acétate d'éthyle)
   - Rf=0,5 (révélation à 254nm, 356nm, I₂).
2°) Spectrométrie de masse (ionisation chimique à l'ammoniac).
   La figure 6 représente le spectre de masse. Le pic à M+1=294 confirme bien la masse moléculaire de 293.
3°) RMN (200MHz).

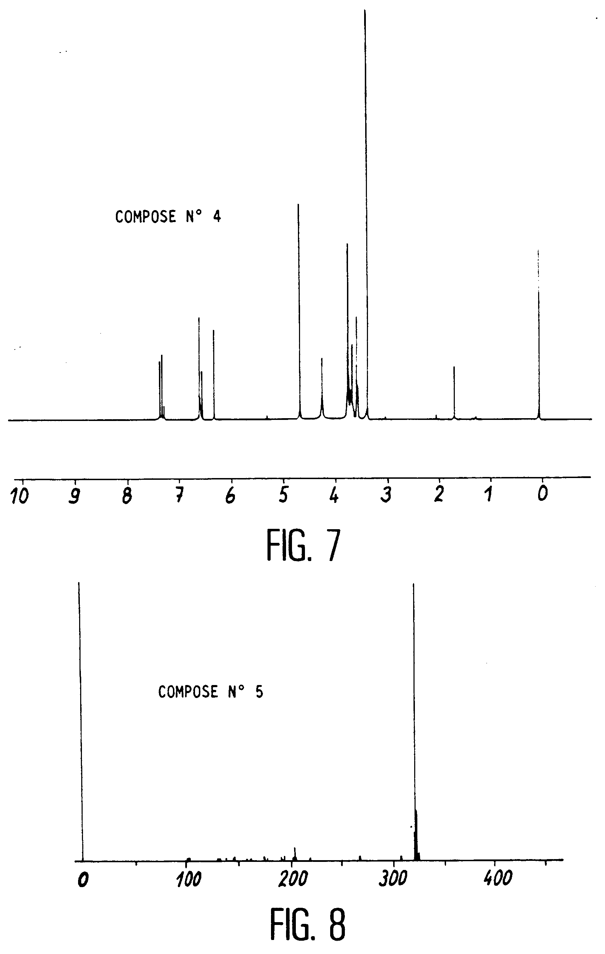

   La figure 7 représente le spectre du proton obtenu et confirme la structure du produit.
4°) Solubilité dans l'eau : 0,4g/l (1,33mM).

### Exemple 4 : Préparation de la 7-diméthylamino-4-(2′,5′,8′-trioxanonyl)coumarine (composé n°5) de formule :

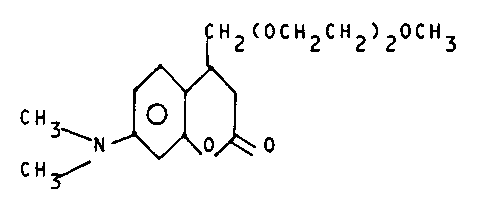

Dans un erlenmeyer de 25ml, on introduit 2,06g (15mmol) de 3-diméthylaminophénol, 3,72g (15mmol) de composé n° 1, 7ml d'éthanol absolu et 2,35g de chlorure de zinc.

On porte à l'ébullition sous agitation pendant 24h et on verse la solution dans un mélange de 200ml de dichlorométhane et de 150ml d'eau. On extrait la phase aqueuse avec deux fois 50ml de dichlorométhane et on lave les phases organiques réunies avec 50ml d'eau. On évapore ensuite jusqu'à siccité.

On purifie le produit obtenu sur colonne de silice en utilisant l'acétate d'éthyle comme éluant.

On obtient ainsi 3,36g du composé n°5, ce qui correspond à un rendement de 70%.

Les caractéristiques du produit sont les suivantes.
1°) CCM (silice fluorescente K₆F, acétate d'éthyle).
   - Rf=0,55 révélé à 254nm, 365nm et à l'iode.
2°) Spectre de masse (ionisation chimique à l'ammoniac).
   La figure 8 représente le spectre obtenu. Le pic à M+1=322 confirme la masse moléculaire de 321.

### Exemple 5 : Préparation de la 7-diéthylamino-4-(2′,5′,8′-trioxanonyl) coumarine (composé n° 6) de formule

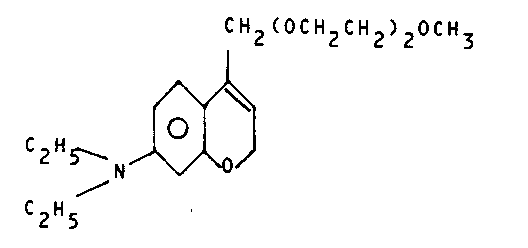

On suit le même mode opératoire que dans l'exemple 4 pour préparer ce composé en partant du composé n° 1, mais en utilisant 2,48g (15mmol) de 3-diéthylaminophénol au lieu des 2,06g de 3-diméthylaminophénol, en purifiant le produit brut sur une colonne de silice, mais en utilisant comme éluant CH₂Cl₂ puis deux mélanges CH₂Cl₂-THF (95:5 et 90:10).

On obtient ainsi 3,1g du composé n°6, ce qui correspond à un rendement de 60%.

Les caractéristiques du produit sont les suivantes.
1°) CCM (silice fluorescente K₆F, butanol-acide acétique, 60:20:20).
   - Rf=0,68 révélé à 254nm, 356nm et à l'iode.
2°) Spectre de masse (ionisation chimique à l'ammoniac)

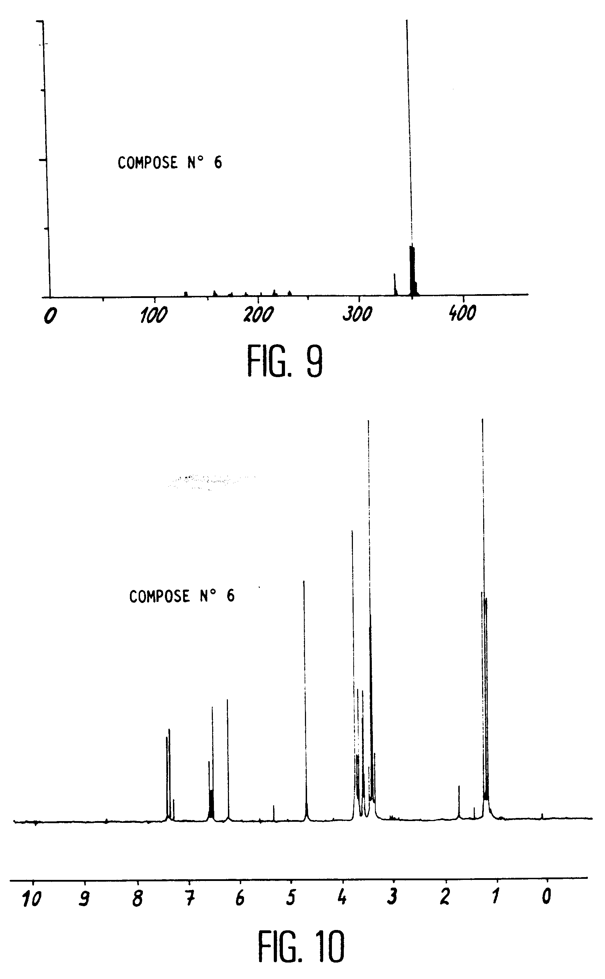

   La figure 9 représente ce spectre. Le pic à M+1=350 confirme la masse moléculaire de 349.
3°) RMN (200MHz)
   La figure 10 représente le spectre du proton qui confirme la structure du composé.

### Exemple 6 : Préparation de 2,3,6,7-tétrahydro-9-(2′,5′,8′-trioxanonyl)-1H, 5H, 11H-(1)-benzopyrano-(6,7,8-ij)-quinolizinone-11 (composé n° 7) de formule :

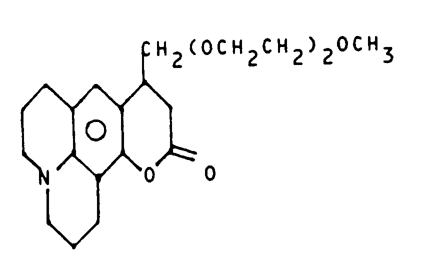

Dans un ballon de 100ml, on introduit 3g de 8-hydroxyjulolidine, 3,93g de 3,6,9-trioxadécanoate d'éthyle, 12ml d'éthanol absolu et 2,49g de chlorure de zinc. On porte à l'ébullition sous agitation pendant 24h, puis on verse le contenu du ballon dans un mélange de 200ml de dichlorométhane et de 150ml d'eau. On extrait la phase aqueuse avec deux fois 50ml de dichlorométhane et on lave les phases organiques réunies avec 50ml d'eau, puis on évapore jusqu'à siccité.

On purifie le produit brut sur une colonne de silice en utilisant comme éluant CH₂Cl₂ puis le mélange CH₂Cl₂-THF (90:10) et on fait cristalliser l'huile obtenue dans de l'eau.

On obtient ainsi 3,09g du composé n°7, ce qui correspond à un rendement de 52%.

Les caractéristiques du produit sont les suivantes :
1°) CCM (silice fluorescente K₆F, CH₂Cl₂-THF, 90:10).
   - Rf=0,6 révélé à 254nm, 356nm et à l'iode.
2°) Spectre de masse (ionisation chimique à l'ammoniac).

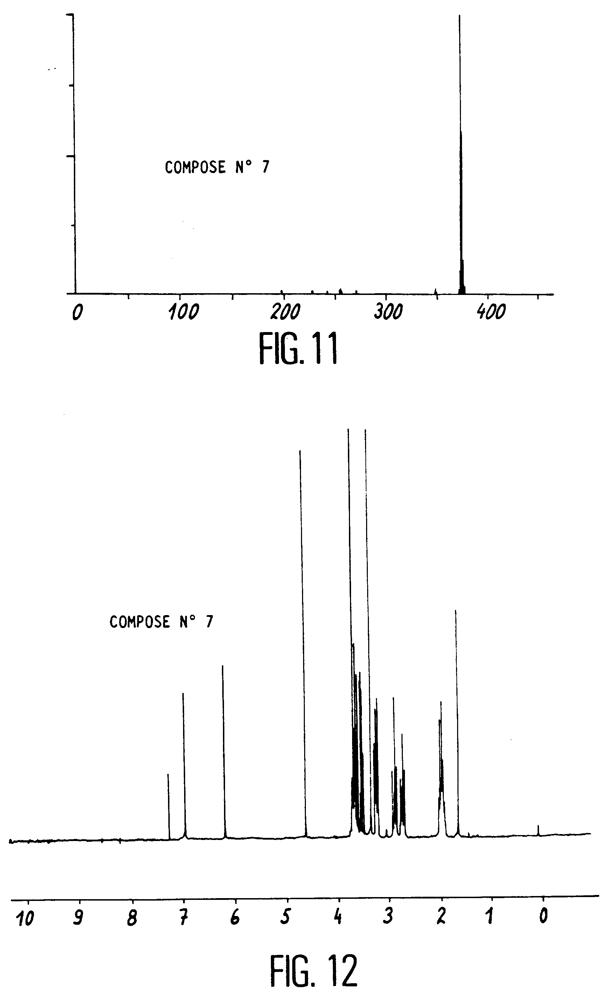

   La figure 11 représente le spectre obtenu. Le pic à M+1=374 confirme bien la masse moléculaire de 373.
3°) RMN (200MHz)
4°) Solubilité dans l'eau : 0,2g/l.
5°) Solubilité dans le mélange éthanol-eau (30:70):3g/l.
   La figure 12 représente le spectre du proton qui confirme bien la structure attendue.

### Exemple 7 : Préparation de la 2,3,6,7-tétrahydro-9-(9′-carboxyéthyl-2′,5′,8′ trioxanonyl)-1H,5H,11H-(1)-benzopyrano-(6,7,8-ij)-quinolizinone-11 (composé n°8) de formule :

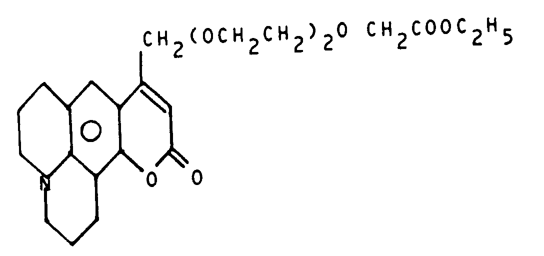

Dans un ballon de 100ml, on introduit 2,23g de 8-hydroxyjulolidine, 4,6g du composé n°2, 13,5ml d'éthanol absolu et 1,85g de chlorure de zinc. On porte à l'ébullition sous agitation pendant 24h, puis on verse le contenu du ballon dans un mélange de 450ml de dichlorométhane et de 170ml d'eau. On extrait la phase aqueuse avec deux fois 50ml de dichlorométhane, et on lave les phases organiques avec 50ml d'eau, puis on évapore jusqu'à siccité.

On purifie le produit brut sur une colonne de silice en utilisant comme éluant CH₂Cl₂ puis CH₂Cl₂ : THF (95:5).

On obtient ainsi 5,25g du composé n° 8, ce qui correspond à un rendement de 60%.

Les caractéristiques du produit sont les suivantes :
1°) CCM (silice fluorescente K₆F, CH₂Cl₂-THF, 95:5)
   - Rf=0,42 révélé à 254nm, 356nm et à l'iode.
2°) Spectre de masse (impact électronique).

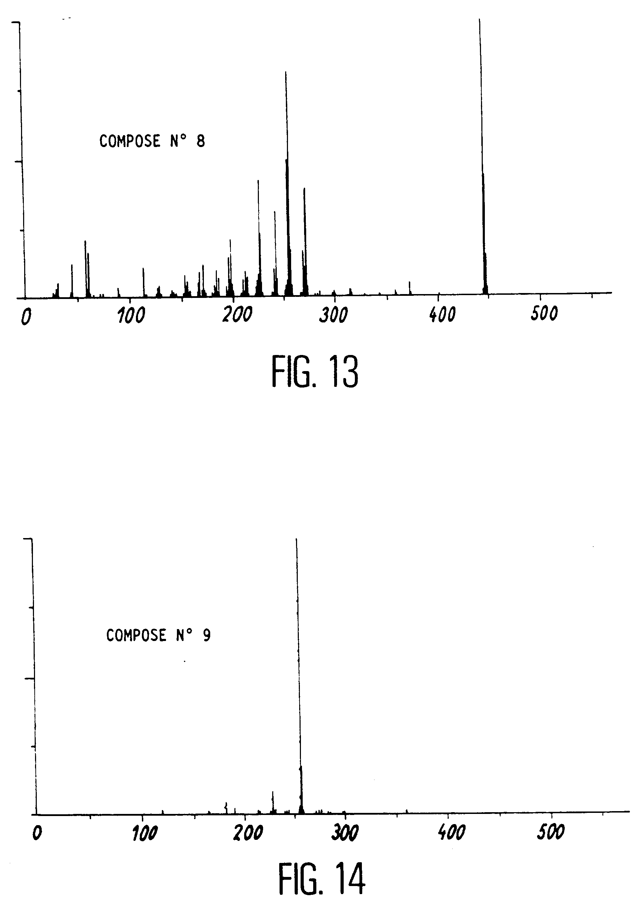

   La figure 13 représente le spectre obtenu. Le pic moléculaire à M=445 confirme bien la masse du produit.

### Exemple 8 : Préparation de la 2,3,6,7-tétrahydro-9-(9′-carboxy-2′,5′,8′-trioxanonyl)-1H,5H,11H-(1)-benzopyrano-(6,7,8-ij)-quinolizinone-11 (composé n°9) de formule

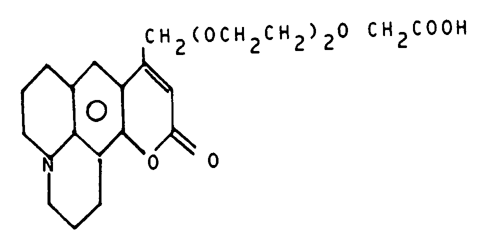

On dissout 3,17g (7,11mmol) du composé n°8 dans 63ml de méthanol et on introduit 95ml d'eau contenant 626mg (15mmol) de soude. On chauffe à 50°C sous agitation pendant 1h à l'abri de la lumière, puis on ramène à 20°C. On acidifie à pH 1,5 avec HCl 3N et on agite durant 30min. On ramène le pH à 6,4 avec de la soude 3N et on agite pendant 30min. On évapore partiellement le méthanol sous vide et on extrait les impuretés avec 200ml de dichlorométhane, puis on évapore à sec sous vide et on reprend le résidu dans 50ml d'éthanol. On filtre les sels minéraux et on met à sec.

On obtient ainsi 2,5g du composé n°9 sous la forme d'une huile se solidifiant lentement, ce qui correspond à un rendement de 84%.

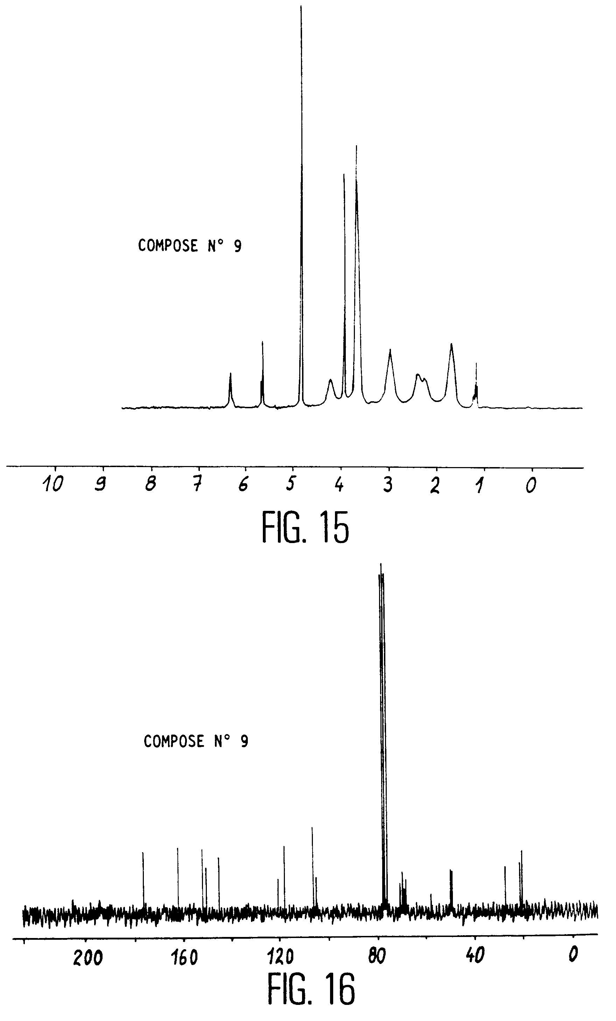

Les caractéristiques de ce produit sont les suivantes.
1°) CCM (silice fluorescente K₆F, butanol - acide acétique - eau 60:20:20).
   - Rf=0,45 révélé à 254nm, 356nm et à l'iode.
2°) Spectre de masse (ionisation chimique à l'ammoniac).
   La figure 14 représente le spectre obtenu. Le pic à M=256 révèle une fragmentation entre le carbone et le premier oxygéne de la chaîne latérale correspondant au substituant R¹.
3°) Spectre RMN
   La figure 15 représente le spectre du proton qui confirme la structure du composé.

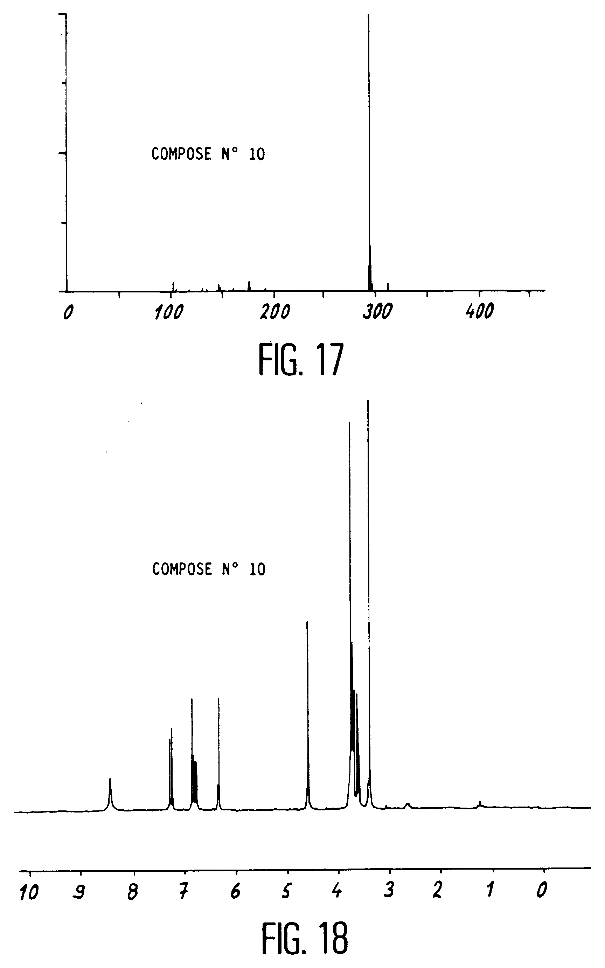

   La figure 16 représente le spectre du carbone 13 de ce composé.

### Exemple 9 : Préparation de la 7-hydroxy-4-(2′,5′,8′-trioxanonyl)-couramine (composé n°10) de formule :

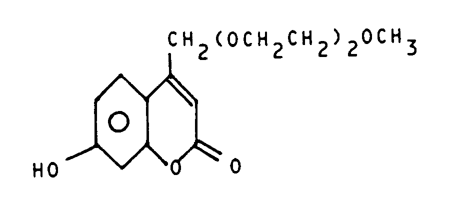

Dans un ballon de 25ml, on introduit 3,3g (0,03mol) de résorcinol, 7,4g (0,03mol) de composé n°1 et 7,5ml d'acide trifluoroacétique. On agite à 75°C pendant 5h, puis on verse le contenu du ballon dans un mélange de 300g d'eau et de glace. On extrait par deux fois 150ml de butanol et on lave les phases butanoliques par deux fois 50ml d'eau, puis on évapore jusqu'à siccité.

On purifie le produit brut par chromatographie sur colonne de silice en utilisant l'acétate d'éthyle comme éluant et on recristallise les fractions les plus pures dans un mélange éthanol-eau (30:70).

On obtient ainsi 2g du composé n°10, ce qui correspond à un rendement de 23%.

Les caractéristiques du produit sont les suivantes :
1°) CCM (silice fluorescente K₆F, acétate d'éthyle).
   - Rf=0,48 révélé à 254nm, 356nm et à l'iode.
2°) Spectre de masse (ionisation chimique à l'ammoniac)
   La figure 17 représente le spectre obtenu. Les pics à 295 (M+1) et à 312 (M+18) confirment la masse moléculaire de 294.
3°) Spectre RMN du proton (200Mhz).
   La figure 18 représente le spectre obtenu qui confirme la structure du composé.
4°) Solubilité dans l'eau : 1g/l.
5°) Solubilité dans le mélange eau-éthanol (90:10) : 7g/l.

### Exemple 10 : Préparation de la 4-(2′-5′,8′-trioxanonyl)-esculétine (composé n°11) de formule :

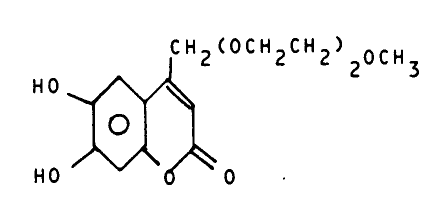

Dans un ballon de 100ml, on introduit 3,05g (12mmol), de triacétoxybenzène (12mmol), 3g (12mmol) du composé n°1, puis 14ml d'acide sulfurique. On porte à 80°C pendant 30min, puis on refroidit et on verse sur un mélange de 50ml d'eau et 50ml de n-butanol. On sépare la phase organique et on la rince avec 4 fois 50ml d'eau, puis on évapore sous vide.

On purifie le produit sur colonne de silice en utilisant comme éluant le mélange dichlorométhane-méthanol (75:25). On évapore les fractions purifiées, on les reprend dans le dichlorométhane, puis on élimine un insoluble et on concentre à sec.

On obtient ainsi 270mg de composé n°11, ce qui correspond à un rendement de 8%.

Les caractéristiques du produit sont les suivantes :
1°) CCM (silice fluorescente K₆F, CH₂Cl₂-MeOH, 75:25).
   - Rf=0,85 révélé à 254nm, 356nm et au chlorure ferrique.
2°) Spectre de masse (ionisation chimique à l'ammoniac).

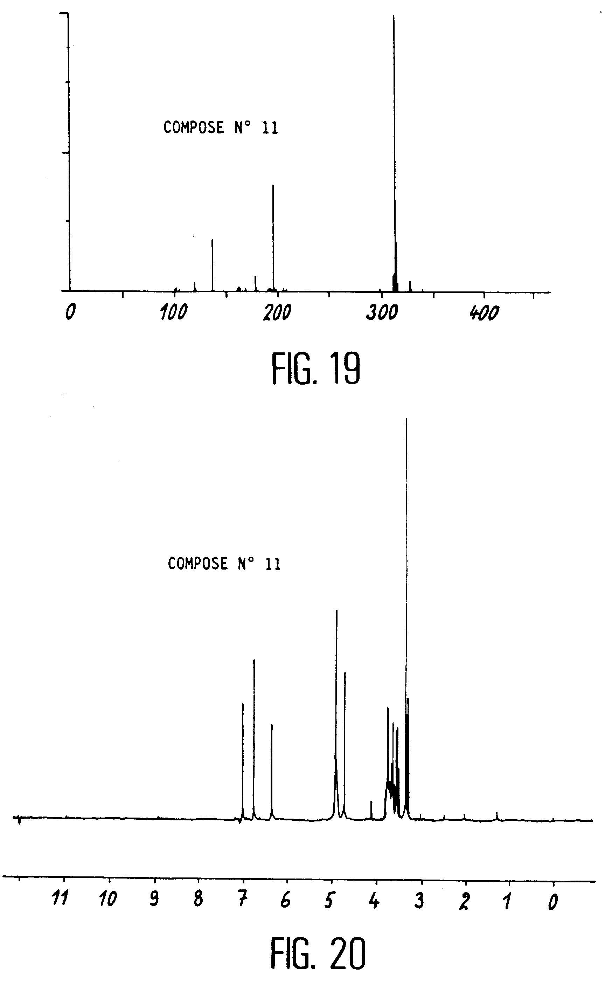

   La figure 19 représente le spectre obtenu. Les pics à 311 (310+1) et 328 (311+17) confirment la masse moléculaire du produit.
3°) Spectre RMN du proton (200MHz).
   La figure 20 représente le spectre obtenu qui confirme la structure du composé.

### Exemple 11 : Préparation du N-carbobenzyloxy-L-leucine-4-(2′,5′,8′-trioxanonyl)-7-coumarinylamide (composé n° 12) de formule :

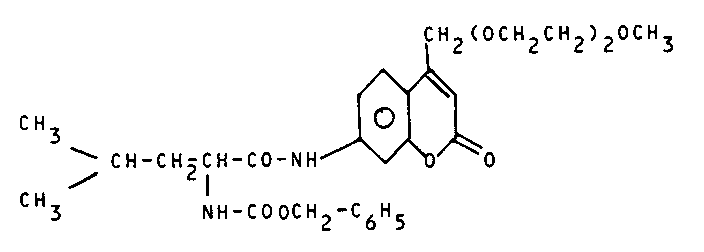

Dans cet exemple, on couple le composé n° 4 avec la N-carbobenzyloxy-L-leucine pour préparer un substrat d'enzyme en utilisant le dicyclohexylcarbodiimide (DCCI).

Dans un erlen de 25ml, on dissout 1,06g (4mmol) de N-carbobenzyloxy-L-leucine et 1,17g (4mmol) du composé n°4 dans 10ml de diméthylformamide. On introduit sous agitation 989mg (4,8mmol) de DCCI et on agite pendant 2h, puis on filtre le précipité formé. On verse sur un mélange de 50ml d'eau et 50ml de dichlorométhane, on lave la phase organique avec 20ml de HCl 1N, 20ml de bicarbonate de sodium à 0,5% et 20ml d'eau. On sèche ensuite la solution et on la concentre à sec.

On obtient ainsi 2g du composé n°12, ce qui correspond à un rendement de 92,6%.

Les caractéristiques du produit sont les suivantes.
1°) CCM (silice fluorescente K₆F, butanol - acide acétique - eau, 60:20:20).
   - Rf=0,72 révélé à 254nm et 356nm.
2°) Spectre de masse (ionisation chimique à l'ammoniac).

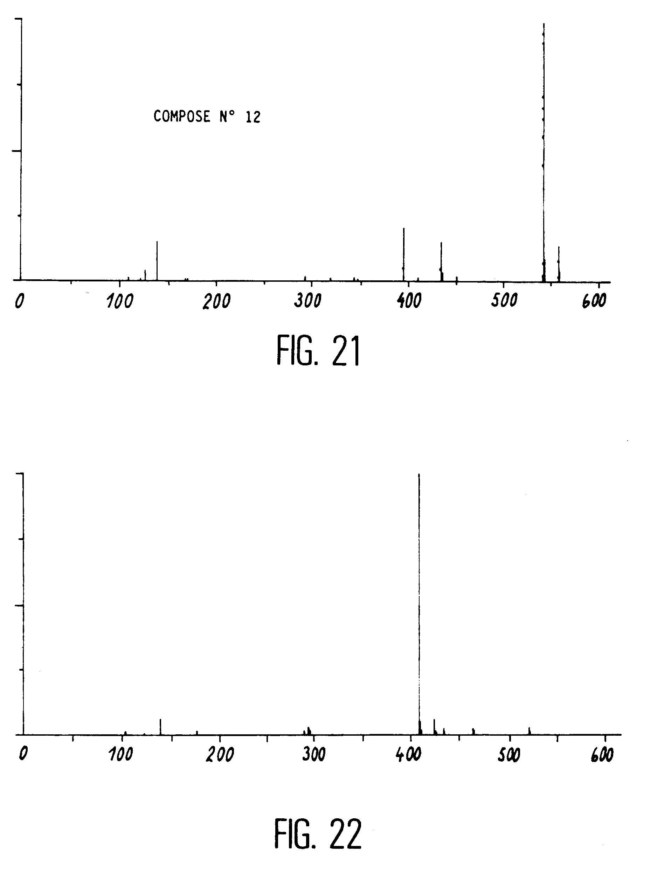

   La figure 21 représente le spectre obtenu. Les pics à 541 (M+1) et à 558 (M+18) confirment bien la masse moléculaire de 540.

### Exemple 12 : Préparation du N-carbobenzyloxy-L-leucine-4-(2′,5′,8′-trioxanonyl)-7-coumarinylamide (composé n°12).

Dans cet exemple, on utilise un autre procédé pour préparer le composé n°12 de l'exemple 11. Dans ce cas, on réalise le couplage du composé n°4 avec la N-carbobenzyloxy-L-leucine après formation intermédiaire d'un anhydride mixte de la N-carbobenzyloxy-L-leucine.

Dans un ballon de 250ml, on introduit à l'abri de l'humidité, 72ml de THF anhydre, 3,62g (13,7mmol) de N-carbobenzyloxy-L-leucine et 1,45g (14,3mmol) de N-méthylmorpholine. On refroidit à -15°C et on introduit 1,96g (14,3mmol) de chloroformiate d'isobutyle et on laisse réagir pendant 5min.

A cette température, on introduit en 45min, 4g (13,7mmol) du composé n°4 dissous dans 144ml de THF. On agite pendant 1h supplémentaire à -15°C, puis à 20°C jusqu'au lendemain. On filtre le produit insoluble et on évapore à sec. On reprend le résidu avec 50ml de dichlorométhane et on le lave avec 20ml de HCl 1N, 20ml de bicarbonate de sodium à 0,5% et 20ml d'eau. Puis on sèche et on concentre à sec.

On obtient ainsi 7,8g du composé n°12, ce qui correspond à un rendement de 91,5%.

Les caractéristiques de ce produit sont les mêmes que celles de l'exemple 11.

### Exemple 13 : Préparation du chlorhydrate de L-leucine-4-(2′,5′,8′, trioxanonyl)-7-coumarinylamide (composé n°13) de formule :

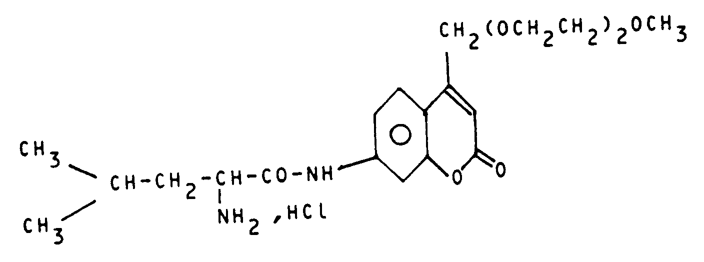

On prépare ce composé à partir du composé n°12 en éliminant tout d'abord le groupement N-carbobenzyloxy de la leucine par hydrogénation catalytique.

Dans une fiole à hydrogéner, on introduit 48ml d'éthanol, 8,5g du composé n°12 et 2,55g de palladium sur charbon à 5%. On purge à l'azote et on introduit de l'hydrogène sous agitation pendant 5h à la pression atmosphérique et à la température ambiante. On filtre le catalyseur et on évapore à sec. On purifie le produit obtenu par chromatographie sur colonne de silice en utilisant comme éluant le THF.

On obtient ainsi 2,7g du composé n°13, ce qui correspond à un rendement de 42%.

On forme ensuite le chlorhydrate correspondant en dissolvant les 2,7g de composé n°13 dans 5,5ml de méthanol et en ajoutant 730µl de HCl concentré. On évapore ensuite sous vide jusqu'à siccité, on ajoute 6ml d'isopropanol et on maintient au froid pendant 24h. On filtre le précipité formé, on recueille encore du produit en diluant le filtrat à l'éther éthylique et on obtient ainsi en tout 2g de chlorhydrate du composé n°13, ce qui correspond à un rendement de 68%.

Les caractéristiques de ce produit sont les suivantes :
1°) CCM
   a) silice fluorescente K₆F, butanol -acide acétique - eau, 60:20:20
      - Rf=0,46 révélé à 254nm, 356nm et à la ninhydrine.
   b) silice fluorescente K₆F, THF.
      - Rf=0,25 révélé à 254nm, 356nm et à la ninhydrine.
2°) Spectre de masse (ionisation chimique à l'ammoniac).
   La figure 22 représente le spectre obtenu. Les pics à 407 (M+1) et 424 (M+18) confirment bien la masse moléculaire de 406.
3°) Spectre RMN.

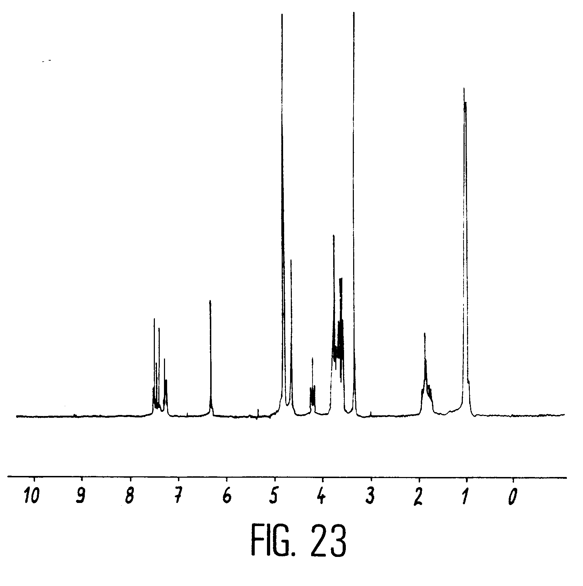

   La figure 23 représente le spectre du proton qui confirme bien la structure du produit obtenu.

### Exemple 14.

Dans cet exemple, on étudie les propriétés du composé n°13 et son utilisation comme substrat hydrosoluble de la leucine aminopeptidase.

### 1. Etudes en cuve de l'activité enzymatique.

Du fait de la présence possible dans le substrat Leu ATC (composé n° 13) d'une trace d'ATC (7-amino-4-(2′,5′,8′-trioxanonyl)-coumarine qui est le produit d'hydrolyse, on a retenu une méthode cinétique pour la détermination de l'activité enzymatique de la leucine aminopeptidase, c'est-à-dire que le signal est constitué par la différence d'intensité de fluorescence au temps 2 minutes et au temps 12 minutes. De cette manière, l'interférence de l'ATC présent avant l'hydrolyse est éliminée.
a) Détermination des longueurs d'onde optimales.
Les spectres d'excitation et d'émission de l'ATC et de la leucine-ATC (composé n°13) ont été tracés à partir de solutions 10⁻⁷M dans le tampon Tris pH 7,8.
Les maxima obtenus sont consignés dans le tableau 1 avec les maxima correspondants de substrats commerciaux dérivés de la 7-amino-4-méthyl-coumarine (Leu-AMC) ou de l'acide 7-amino-4-coumarinyl-méthanesulfonique (GlyGly Arg AMCS).

**TABLEAU 1**

| Longueurs d'onde maximales d'excitation/fluorescence et de mesure (en nm) pour les dérivés de l'ATC, l'AMC et l'AMCS. | | | |
|---|---|---|---|
| | Substrat (nm) | Produit d'hydrolyse (nm) | Mesure (nm) |
| Composé n°13 (Leu ATC) | 330/405 | 350/465 | 370/480 |
| Leu AMC | 327/390 | 345/440 | 380/440 |
| GlyGly Arg AMCS | 336/409 | 362/462 | 380/462 |

La position des spectres d'excitation et de fluorescence du composé n°13 (Leu-ATC) et du produit d'hydrolyse correspondant (ATC) est intermédiaire entre celle des dérivés de l'AMC et de l'AMCS, tandis que son déplacement de Stokes est le plus large.
Aux longueurs d'onde utilisées pour la mesure, le rapport des intensités de fluorescence du produit d'hydrolyse et du substrat à concentration équimolaire est de l'ordre de 400 pour L'ATC, 300 pour l'AMC et 250 pour l'AMCS. Ainsi, la fluorescence du substrat n'interfère aucunement avec celle du produit d'hydrolyse.
b) Détermination des caractéristiques de la réaction enzymatique.
La vitesse initiale de la réaction a été étudiée pour une concentration en substrat constitué par le composé n° 13 (Leu-ATC) allant de 2x10⁻⁵mol/l à 2x10⁻³mol/l en utilisant les réactif suivants.
- Chlorhydrate de Leu-ATC cristallisé de l'exemple 13 : solutions aqueuses 4x10⁻⁵ à 4x10⁻³mol/l,
- Leucine aminopeptidase (Sigma, réf N5006) : solution aqueuse à 7,53µg/ml dans le tampon Tris 0,2M pH7,8,
- Tampon Tris :
   solution A : Tris 0,2M soit 24,23g/l
   solution B : HCl 0,1M

Ce tampon est préparé en mélangeant 25ml de solution A et 33,7ml de solution B et en complétant à 100ml avec de l'eau. Le pH doit être à 7,8, - Solution d'acide perchlorique 1,17 N (dilution de l'acide pur au 1/10).
Pour cette détermination, on prépare tout d'abord dans des tubes la gamme suivante de concentrations en substrat (composé n°13, Leu-ATC) :
0 - 2 - 4 - 10 - 20 - 50 - 100 - 200 x 10⁻⁵M (concentration finale).
On ajoute à chaque tube une concentration de 1,51 µg/ml en leucine aminopeptidase, l'ensemble étant en solution dans le tampon Tris à pH 7,8 préchauffé à 37°C et les tubes étant dans un bain marie thermostaté à 37°C. Aprés 2min, 250µl sont prélevés dans chaque tube et versés dans des tubes contenant 250µl de solution perchlorique et 750µl d'eau. La même opération est répétée à 6min. !'ensemble des tubes est dilué au millième. L'intensité de fluorescence IF est mesurée à 370/480nm. La différence des intensités à 6 et 2min : IF(6)-IF(2), constitue le signal.
La concentration en ATC correspondante est calculée par rapport à une gamme d'étalonnage allant de 10⁻⁹ à 10⁻⁸M. Les résultats sont donnés dans le tableau 2 qui suit :

**TABLEAU 2**

| | | | | | | | | |
|---|---|---|---|---|---|---|---|---|
| Concentration en Leu-ATC (en 10⁻⁵M) | 0 | 2 | 4 | 10 | 20 | 50 | 100 | 200 |
| IF(6)-IF(2) | 0 | 5,5 | 11,5 | 21 | 38,5 | 45 | 47 | 48 |
| Concentration en ATC dans solution de lecture (en nM) | 0 | 1,38 | 2,88 | 5,25 | 9,63 | 11,25 | 11,75 | 12,0 |
| Concentration en ATC dans solution d'hydrolyse (en uM/min) | 0 | 1,7 | 3,6 | 6,6 | 12,0 | 14,1 | 14,7 | 15,0 |

La constante de vitesse kcat est calculée par le rapport Vm/Ez où Vm est la vitesse maximale en µmol/s / mg Ez et Ez est la quantité d'enzyme contenue dans un mg, exprimée en µmol. La masse molaire de la leucine aminopeptidase est prise égale à 300 000.

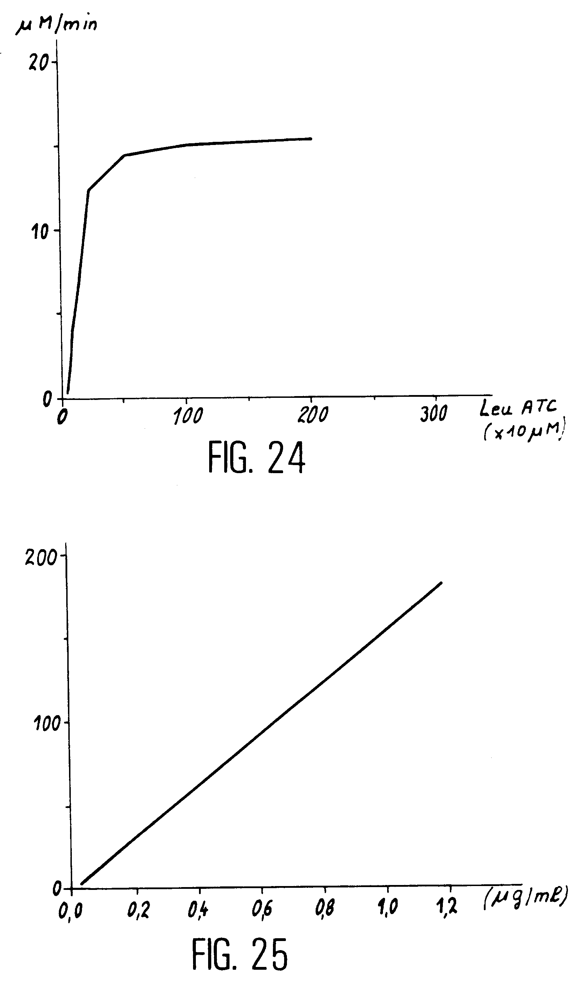

La figure 24 est une courbe illustrant l'évolution de la vitesse initiale (en µM/min)en fonction de la concentration en substrat (Leu-ATC) exprimé en µM.
Cette courbe présente une allure hyperbolique caractéristique d'une cinétique michaelienne. Les points expérimentaux ont été ajustés à l'équation correspondante par régression non linéaire (logiciel Graphpad) et ont permis de déterminer la constante d'affinité, Km, la vitesse maximale Vm et la constante de vitesse kcat (rapport de la vitesse maximale à la concentration en enzyme). Les résultats, comparés à ceux des substrats connus, sont rapportés dans le tableau 3.

**TABLEAU 3**

| | Km (mM) | Vm (µmol/mgEz/min) | kcat (s⁻1) |
|---|---|---|---|
| Composé n° 13 Leu ATC | 0,11 | 10,3 | 51,5 |
| Leu AMC | 0,16 | 4,0 | |
| Leu-β naphtylamide | 0,15 | 5,5 | |

Il apparaît que l'affinité de la LeuATC pour la leucine aminopeptidase est meilleure que celle des deux autres substrats tandis que la vitesse maximale est plus élevée. Ceci est peut-être à rapporter au fait que le milieu réactionnel ne contient aucun solvant organique, contrairement aux autres substrats. De plus, la comparaison de la Leu ATC et de la Leu AMC montre que la présence de la chaîne latérale hydrosoluble R¹ ne pénalise pas la réaction enzymatique, ce qui est un avantage par rapport aux substrats concurrents dérivés de l'acide 7-amino-4-coumarinyl-méthanesulfonique. Dans ceux-ci, le groupement sulfonique hydrosoluble diminue fortement la vitesse maximale et l'affinité.
c) Détermination des caractéristiques analytiques de la réaction.
Les conditions de réaction ont été déterminées d'après l'étape précédente. La concentration en substrat Leu-ATC a été choisie pour donner une vitesse maximale (1mM). Le temps de réaction a été porté à 12 minutes, en mesurant la différence des intensités de fluorescence aux temps 12 et 2 minutes. Pour évaluer la limite de quantification, mesurer la répétabilité et tester la linéarité, 6 gammes d'enzymes ont été réalisées, contenant des concentrations de 0 - 0,05 - 0,10 - 0,25 - 0,50 - 1µg/ml, en utilisant les mêmes réactifs que précédemment. On obtient ainsi la gamme suivante :

| Ez (mg/ml) | 0 | 0,05 | 0,10 | 0,25 | 0,50 | 1 |
|---|---|---|---|---|---|---|
| Leu-ATC (4x10⁻³M) | 250 | 250 | 250 | 250 | 250 | 250 |
| Tampon Tris | 750 | 740 | 730 | 700 | 650 | 550 |
| Enzyme 5µg/ml | 0 | 10 | 20 | 50 | 100 | 200 |

Pour effectuer l'évaluation, on prépare une série de tubes contenant 750µl d'eau + 250µl de solution perchlorique 1,17N et au temps 2min, on y tranfère 250ul de chaque échantillon de la gamme d'enzymes. On fait de même au temps 12min. On dilue ensuite tous les tubes au 1/200 dans l'eau et on lit la fluorescence (370/480nm) avec un gain de 2 sur le fluorimètre Perkin Elmer LS3B.
Les résultats obtenus sont donnés sur la figure 25 qui représente la courbe d'étalonnage moyenne illustrant la variation de l'intensité de fluorescence en fonction de la concentration en enzyme (µg/ml).
Cette courbe d'étalonnage a été déterminée par régression linéaire. La moyenne ± écart-type est de 153±7,36 pour la pente, et de -3,10±1,16 pour l'intercept.
Le test de linéarité est significatif au seuil de 5% (F=0,554).
La limite de quantification, calculée par le rapportσ 3 b/à, est de 0,02µg/ml.
La répétabilité, évaluée par le coefficient de variation, est de 17% à 0,05µg/ml, 8% à 0,25µg/ml, et 4% à 1µg/ml.
La limite de détection est donc plus favorable que celle obtenue avec la Leu AMC qui est de 0,03µg/ml.

### Exemple 15 : Préparation du chlorhydrate de N-carbobenzyloxy-L-arginyl-4-(2′,5′,8′ trioxanonyl)-7-coumarinylamide (composé n° 14) de formule :

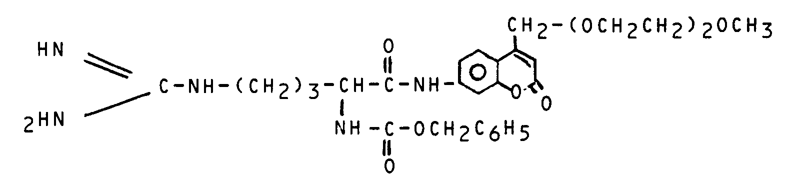

Dans cet exemple, on couple le composé n°4 avec la N-carbobenzyloxy L-arginine, en utilisant le dicyclohexylcarbodiimide (DCCI) comme agent de couplage.

Dans un ballon de 100ml, on dissout 3,45g (10mmol) de chlorhydrate de N-carbobenzyloxy-arginine et 1,467g (5mmol) du composé n°4 dans 15ml de diméthylformamide. On introduit sous agitation 1,05g (5mmol) de DCCI et on agite durant 20h. On filtre et on élimine le précipité.

On évapore le solvant sous vide, puis on reprend par un mélange de 25ml d'acétate d'éthyle et de 2,5ml de méthanol. L'huile qui se sépare est reprise par un mélange de 1,5ml de DMF et de 5ml de méthanol, sur lequel on additionne lentement 60ml d'acétate d'éthyle. Le précipité gommeux formé se solidifie en dessicateur sous vide : 2,14g.

Le produit brut obtenu est purifié sur une colonne de silice en utilisant comme éluants des mélanges ternaires dichlorométhane/méthanol/acide acétique 85/15/5 puis 85/20/5.

Sur les 34 fractions de 10ml recueillies, on concentre les fractions 19 à 30. Le concentrat repris 2 fois dans l'éthanol et réévaporé est additionné d'éther et mis sous agitation. Le produit est filtré et séché sous vide.

Rendement : 605mg (19,5%).

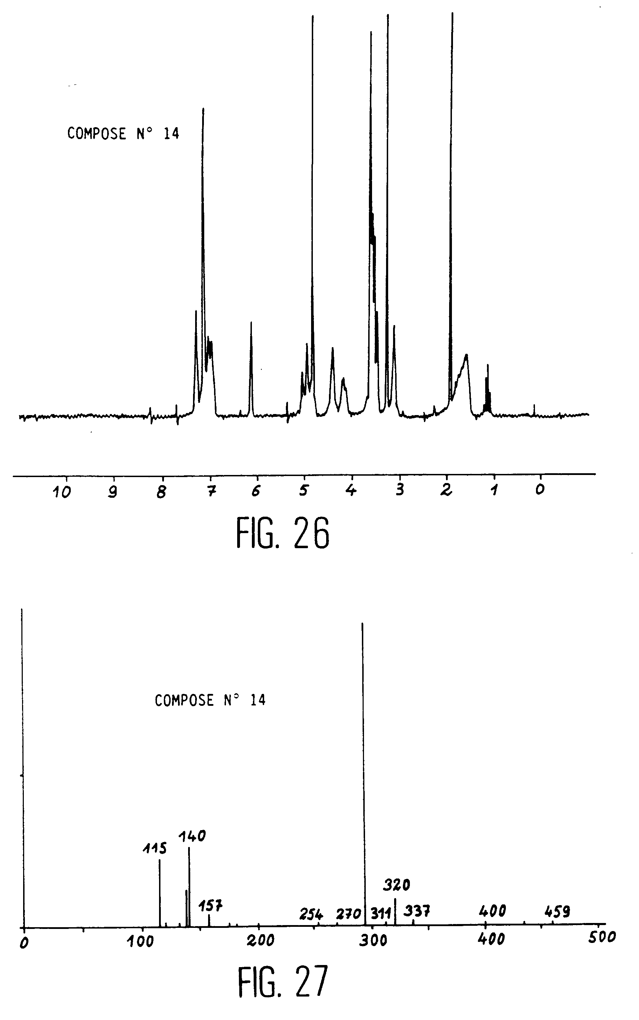

Les caractéristiques du produit sont les suivantes :
1°) C.C.M.
   Silice fluorescente K6F ; solvant d'élution: dichlorométhane / méthanol / acide acétique, 85/20/5.
   Révélation : à 254nm et 356nm (spot bleu-violet)
   Rf : 0,63.
2°) Spectre RMN : la figure 26 représente le spectre du proton qui confirme bien la structure du produit obtenu et montre une solvatation avec une molécule d'acide acétique.
3°) Spectre de masse (ionisation chimique à l'ammoniac).
   La figure 27 représente le spectre obtenu. La molécule est fortement fragmentée, le pic principal à 294 (293+1), représentant la molécule du composé 4, partie la plus stable.

### Exemple 16.

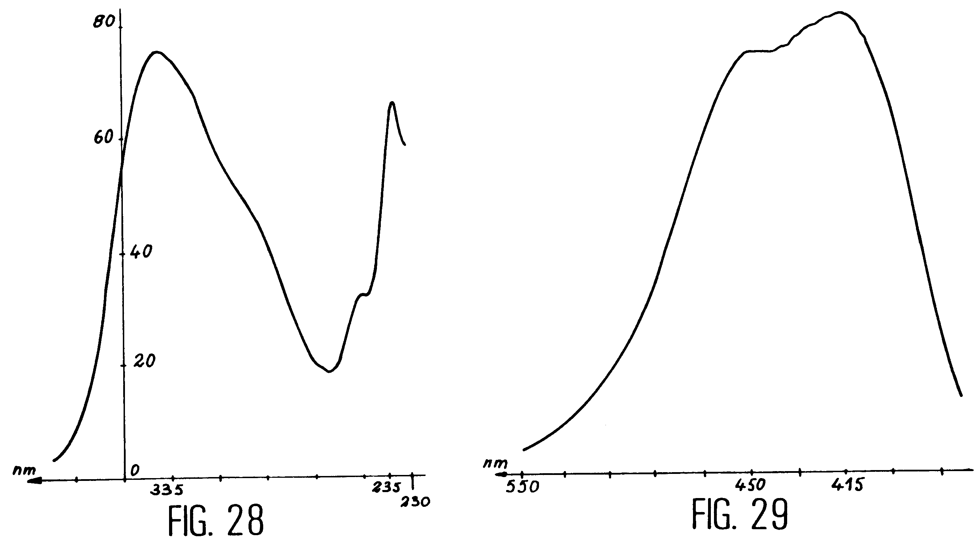

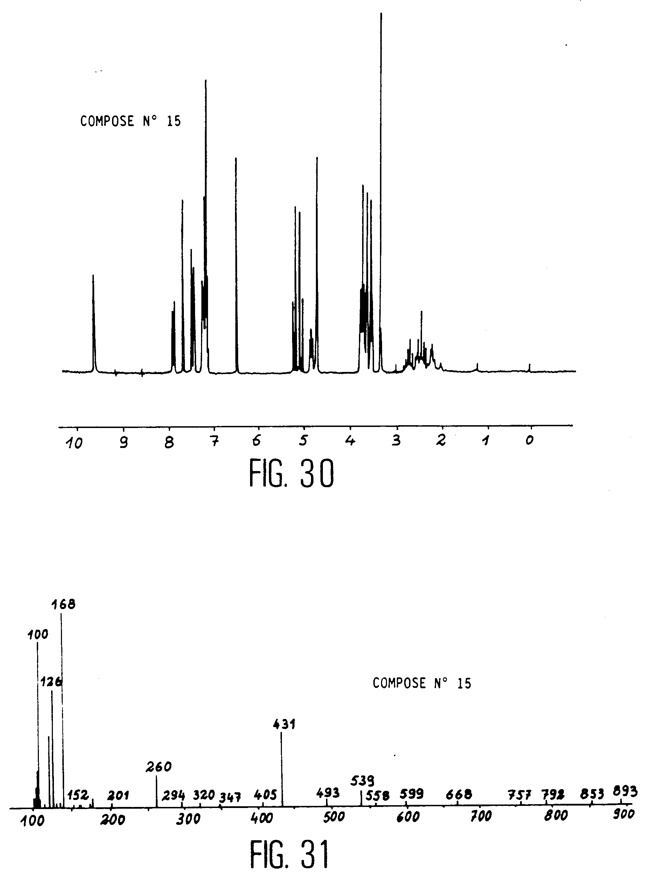

Dans cet exemple, on étudie l'hydrolyse de la Z-Arg-ATC (composé n° 14) par la trypsine.
1°) Détermination des longueurs d'onde optimales.
Les spectres d'excitation et d'émission de l'ATC et de la Z-Arg-ATC ont été tracés à partir de solutions 10⁻⁷ M dans le tampon Tris pH 8 50mM contenant 10mM de CaCl₂.
Les spectres d'excitation et d'émission de la Z-Arg-ATC sont représentés respectivement sur les figures 28 et 29.
Les maxima obtenus sont indiqués dans le tableau 4 avec les maxima correspondants de la Leu-ATC.

**TABLEAU 4**

| Longueurs d'onde (en nm) des maxima d'excitation / flourescence et de mesure pour les dérivés de l'ATC. | | | |
|---|---|---|---|
| | Substrat (nm) | Produit (nm) | Mesure (nm) |
| Leu - ATC | 330/405 | 350/465 | 370/480 |
| Z-Arg-ATC | 335/415 et 450 | 350/465 | 380/490 |

La présence de l'Arg à la place de la Leu sur l'amine de l'ATC entraîne un léger déplacement des spectres vers le rouge (effet bathochrome) qui impose un changement des longueurs d'onde de mesure dans la même direction, afin d'éviter l'interférence du substrat dans la fluorescence du produit.
2°) Caractéristiques de la réaction enzymatique.
a) Réactifs :
   - chlorhydrate de Z-Arg-ATC cristallisé (Seratec) : solution 10⁻³M dans le tampon Tris pH 8,0 + Cl₂Ca 10mM.
   - Trypsine de pancréas de porc (SIGMA T0134) : solution 10⁻³M dans le tampon Tris pH 8,0 + Cl₂Ca 10mM.
   - Tampon Tris :
      solution A : Tris 0,2M soit 24,23g/l + CaCl₂ 40mM soit 5,88g/l
      solution B : HCl 0,1M

      Ce tampon est préparé en mélangeant 250ml de solution A avec 268ml de B et en complétant à 1l avec de l'eau.
   - Tampon acétate :
      solution A : acide acétique 0,02M
      solution B : acétate de sodium 0,02M

      Ce tampon est préparé en mélangeant volume à volume les solutions A et B pour obtenir un tampon de pH 4,75.
   - Solution aqueuse d'acide perchlorique 1,17N (dilution de l'acide pur au 1/10).
b) Procédure
   La gamme de concentrations suivantes en substrat a été préparée dans des tubes :
   0 - 0,2 - 0,5 - 1 - 2 - 5 - 10 x 10⁻⁴ M (concentration finale)

On ajoute à chaque tube une concentration de 6,67µg/ml en trypsine, l'ensemble étant en solution dans le tampon Tris pH 8,0 + CaCl₂ 10mM, à température ambiante. L'enzyme est ajoutée à l'instant 0. Après 2 minutes, 250µl sont prélevés dans chaque tube et versés dans des tubes contenant 250µl d'acide perchlorique 1,17N et 500µl d'eau. Les tubes sont centrifugés puis la solution est diluée au 1/250 dans le tampon acétate. La même opération est répétée à 17 min. L'intensité de fluorescence est mesurée à 380/490nm. La différence des intensités à 17 et 2 min constitue le signal.
La concentration d'ATC correspondante est calculée par rapport à une gamme d'étalonnage allant de 5x10⁻⁹ à 2x10⁻⁷M.
Les résultats sont donnés dans le tableau 5 qui suit.

**TABLEAU 5**

| | | | | | | | |
|---|---|---|---|---|---|---|---|
| \|Arg-ATC\| x 10⁻⁴ M | 0 | 0,2 | 0,5 | 1 | 2 | 5 | 10 |
| I_{F}(17)-I_{F}(2) | 0 | 3,7 | 7,7 | 16,9 | 29,8 | 38,9 | 42,4 |
| \|ATC\| nM solution de lecture | 0 | 3,36 | 7,00 | 15,4 | 27,1 | 35,4 | 38,6 |
| \|ATC\| µM/min solution d'hydrolyse | 0 | 0,224 | 0,467 | 1,03 | 1,81 | 2,36 | 2,57 |

On détermine ensuite Kₘ, Vₘ, kcat et le rapport kcat/Km. Les résultats obtenus sont donnés dans le tableau 6. Dans ce tableau, on a donné également à titre comparatif les valeurs obtenues avec Z-L-Arg-AMC et B_{z} DL Arg-AMC et B_{z}-L-Arg-AMC

**TABLEAU 6**

| | Km (mM) | Vm (nmol/min/mg Ez) | kcat (s⁻¹) | kcat/Km M⁻¹ s⁻¹ |
|---|---|---|---|---|
| Z-L-Arg-ATC | 0,195 | 474 | 0,19 | 975 |
| Bz-L-Arg-AMC | 0,11 | - | 0,42 | 3800 |
| Z-L-Arg-AMC | 0,20 | - | 0,89 | 4500 |
| Bz-DL-Arg-AMC | 0,25 | - | 0,20 | 800 |

On remarque ainsi que le Km est de 0,195mM et le Vm de 3,16µM/min.

La vitesse maximale est de 474nmol/min/mgEz, correspondant à un kcat=0,19s⁻¹ et un rapport kcat/Km=975M⁻¹s⁻¹.

Le substrat Z-L-Arg ATC est donc comparable au Bz-DL-Arg-AMC du point de vue de son affinité et de son activité. En revanche, la vitesse maximale est environ 4 à 5 fois inférieure à celle de la Z-L-Arg AMC. Cette différence n'est pas considérable et peut être compensée par une durée de mesure un peu plus longue.

### 3. Caractéristiques analytiques

Six gammes d'étalonnage contenant une concentration finale en enzyme de 0 - 0,42 - 0,84 - 1,67 - 3,33 - 6,67µg/ml ont été réalisées.

Chaque courbe d'étalonnage a été déterminée par régression linéaire. La moyenne ± écart type est de 2,45x10⁻³ ± 0,003 x 10⁻³
pour la pente et 4,80 x 10⁻³ ± 2,53 x 10⁻³ pour l'intercept. Le test de linéarité est significatif au seuil de 5% (W = 0,245). La limite de quantification, calculée par le rapport 3σ_{b}/a, est de 0,05µg/ ml. La répétabilité, évaluée par le coefficient de variation, est de 9,4% à 0,42µg/ml, 5,9% à 0,84µg/ml, et 1,3% à 6,67µg/ml. La limite de détection de la trypsine par la Z-L-Arg ATC est donc du même ordre que celle obtenue avec les substrats dérivés de l'AMC.

### Exemple 17 : Préparation du N-carbobenzyloxy pyroglutamyl-4-(2′,5′,8′ trioxanonyl)-7-coumarinylamide (composé n° 15).

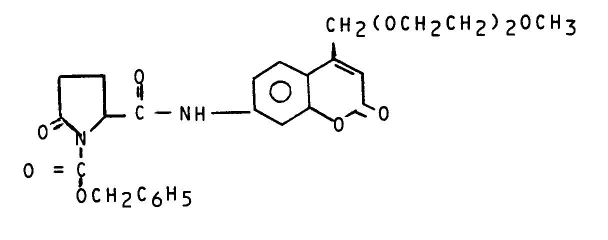

Dans cet exemple, on couple le composé n° 4 avec l'acide N-carbobenzyloxy pyroglutamique, en utilisant le dicyclohexylcarbodiimide (DCCI) comme agent de couplage.

Dans un ballon de 100ml, on dissout 2,24g (8,5mmol) d'acide N-carbobenzyloxy pyroglutamique et 2,5g du composé n° 4 (8,5mmol) dans 30ml de diméthylformamide. On introduit sous agitation 1,76g (8,5mmol) de DCCI et on agite durant 20h. On filtre et on élimine le précipité.

On verse le filtrat sur un mélange de 150 ml d'eau et de 150ml de dichlorométhane. On lave la phase organique par 4x100ml d'acide chlorhydrique 1N, 100ml d'eau, 100ml de bicarbonate à 5% et 50ml d'eau.

La phase organique est séchée sur sulfate de magnésium, et évaporée sous vide. Le résidu est cristallisé dans 20ml d'acétate d'éthyle.

Rendement- = 2,5g (56%),

Les caractéristiques du produit sort les suivantes :
1°) C.C.M. silice fluorescente K6F ; solvant d'élution : éthanol / cyclohexane / THF 30/70/5. Révélation à 254nm et 356nm (spot bleu violet) Rf = 0,16.
2°) Spectre RMN : la figure 30 représente le spectre du proton qui confirme bien la structure du produit obtenu.
3°) Spectre de masse : (ionisation chimique à l'ammoniac). La figure 31 représente le spectre obtenu. Le pic à 539 représente bien le pic moléculaire +17.

### Exemple 18 : Préparation du pyroglutamyl-4-(2′,5′,8′ trioxanonyl)-7-coumarinylamide (composé n° 16).

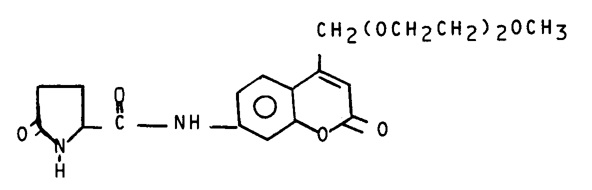

Dans cet exemple, on hydrogène le composé n° 15 en solution dans le tétrahydrofuranne.

Dans une fiole à hydrogéner, on introduit 70ml de tétrahydrofuranne, 2,4g du composé n° 15 et 360mg de palladium sur charbon à 10%. On purge à l'azote et on introduit de l'hydrogène ; on met sous agitation pendant 5h à la pression atmosphérique et à la température ambiante. Après avoir filtré le catalyseur, on évapore à sec, et on recristallise dans 40ml d'acétate d'éthyle.

Rendement : 1,32g (74,5%).

Les caractéristiques du produit sont les suivantes :
1°) C.C.M. :
   Silice fluorescente K6F ; solvant d'élution: dichlorométhane méthanol 90/10 ; révélation à 254nm et 356nm (spot bleu violet) - Rf=0,39.
2°) Spectre de masse : (ionisation chimique à l'ammoniac)

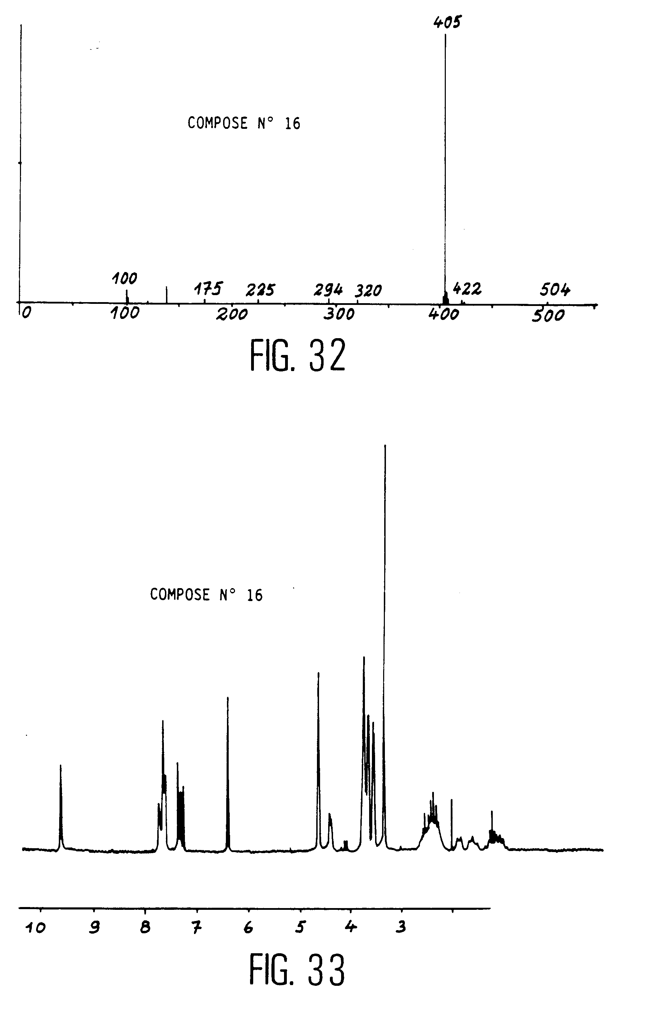

   La figure 32 représente le spectre obtenu. Le pic à 405 représente le pic moléculaire ; le pic à 422 représente le pic moléculaire + 17 ;
3°) Spectre RMN ; La figure 33 représente le spectre du proton qui confirme bien la structure du produit obtenu.

### Exemple 19 : Préparation de N-carbobenzyloxy-α-benzylester γ-glutamyl-4-(2′,5′,8′ trioxanonyl)-7-coumarinylamide de formule (composé 17) :

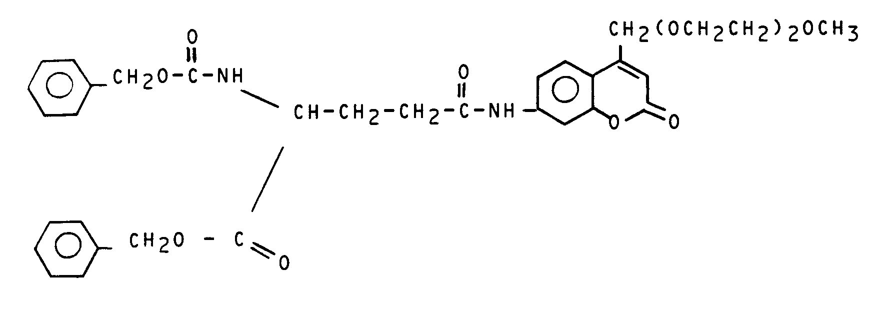

Dans cet exemple, on couple le composé n° 4 avec l'ester α-benzylique de l'acide N-carbobenzyloxyglutamique.

Dans un ballon de 100ml, on dissout 7,43g (0,02mol) d'ester α-benzylique de l'acide N-carbobenzyloxy glutamique, 3,24g d'hydroxybenzotriazole, 5,87g (0,02mol) de composé n°4 dans 55ml de diméthylformamide. On refroidit à 5°C et on introduit sous agitation 4,13g de dicyclohexylcarbodiimide (DCCI) (0,02mol). Après avoir maintenu une heure à 5°C, on agite durant 20h à la température ambiante. On filtre et on élimine le précipité.

On évapore le solvant sous vide, et on reprend par 135ml d'une solution de bicarbonate à 5% et 200ml d'acétate d'éthyle. La phase organique est lavée par 3x135ml d'acide chlorhydrique 1N, 135ml de solution de bicarbonate à 5% et 135ml d'eau.

La solution organique est mise à sec et le produit est purifié sur colonne de silice, avec de l'acétate d'éthyle comme éluant. Les fractions 41 à 77 conduisent à 3,87g (30%) de produit pur.

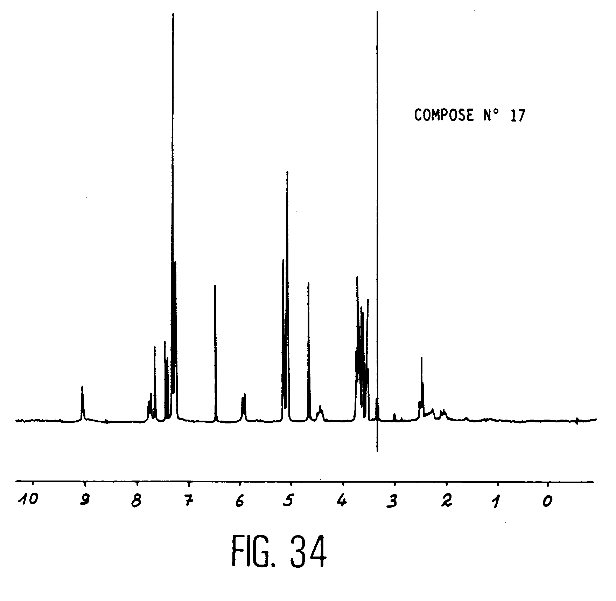

Les caractéristiques du produit sont les suivantes :
1°) C.C.M. :
   Silice fluorescente K6F ; solvant d'élution: acétate d'éthyle. Révélation : à 254nm et 356nm (spot bleu violet) Rf = 0,45.
2°) Spectre RMN : la figure 34 représente le spectre du proton qui confirme bien la structure du produit attendu.

### Exemple 20 : Préparation de γ-glutamyl-4-(2′,5′,8′ trioxanonyl)-7-coumarinylamide (composé n° 18) de formule :

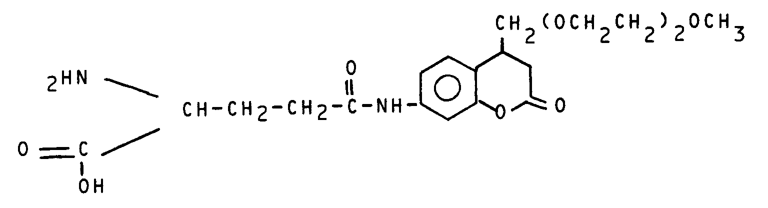

Dans cet exemple, on hydrogène le composé n° 17 en solution dans le tétrahydrofuranne.

Dans une fiole à hydrogéner, on introduit 114ml de tétrahydrofuranne, 3,82g du composé n° 17 et 764mg de palladium sur charbon à 10%. On purge à l'azote et on introduit de l'hydrogène. On met sous agitation pendant 8h à pression atmosphérique et à la température ambiante. Après avoir filtré le catalyseur, on extrait le produit qui a cristallisé dans le catalyseur par 2 x 400ml de méthanol à ébullition.

Le méthanol est concentré sous vide jusqu'à un volume de 100ml. Le produit qui cristallise est centrifugé, rincé une fois au méthanol et séché sous vide.

Rendement : 1,55g (65%).

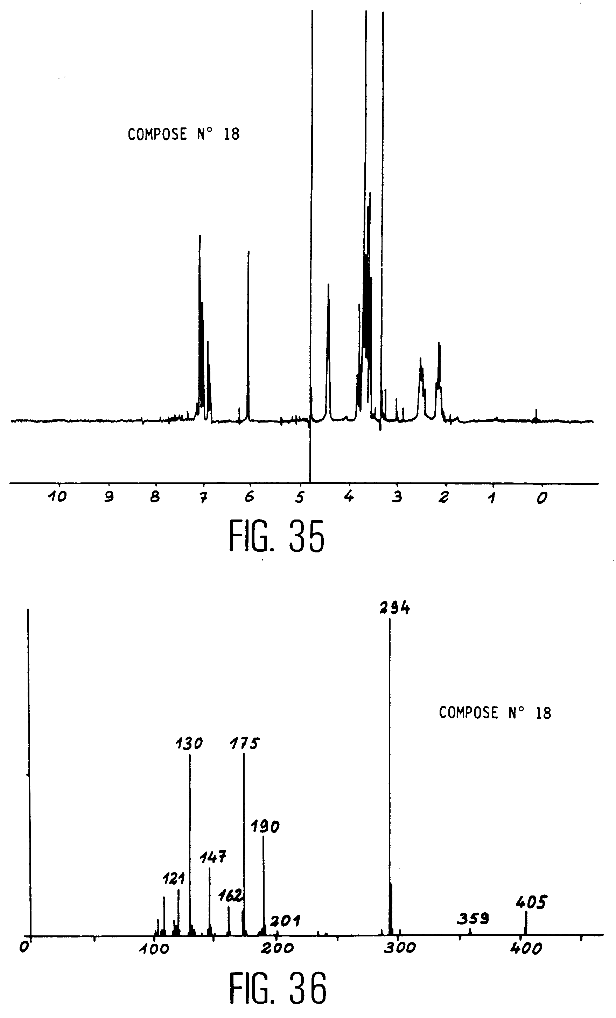

Les caractéristiques du produit sont les suivantes :
1°) C.C.M. : silice fluorescente K6F; solvant d'élution butanol / acide acétique/eau 60/20/20. Révélation à 254nm et 356nm (spot bleu violet). Rf = 0,35 (révélé à la ninhydrine).
2°) Spectre R.M.N. : la figure 35 représente le spectre du proton qui confirme bien la structure du produit attendu.
3°) Spectre de masse (ionisation chimique à l'ammoniac).
   La figure 36 représente le spectre du produit obtenu. On retrouve bien le pic (M+1)=405 de la molécule et le pic à (293+1) du produit 4 (fragmentation au niveau de l'amide).

### Exemple 21 : Préparation du ω-Fmoc α-Boc L-lysyl-4-(2′,5′,8′, trioxanonyl)-7-coumarinyl-amide (composé n°19).

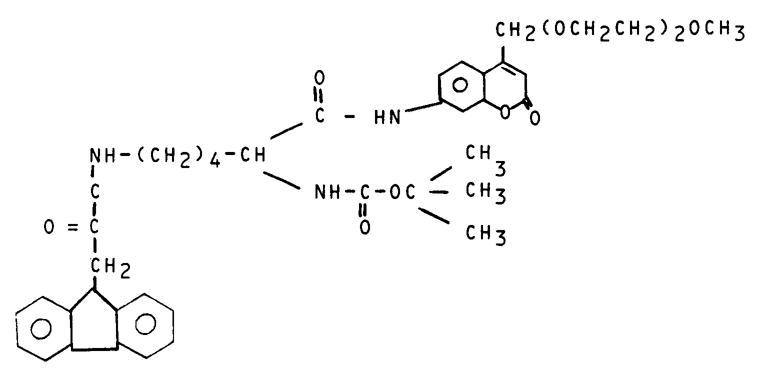

Dans cet exemple on couple le composé n°4 avec le ω-Fmoc αBoc L-leucine, en utilisant le dicyclohexylcarbodiimide (DCCI) comme agent de couplage.

Dans un ballon de 50ml, on dissout 2g (4,27 mmol) de ω-Fmoc α-Boc-L-lysine et 1,25g (4,27mmol) de composé n°4 dans 15ml de diméthylformamide. On introduit sous agitation 0,881g (4,27mmol) de DCCI et on agite durant 20h. On filtre et on élimine le précipité.

On évapore le solvant sous vide. On reprend le résidu par 40ml de dichlorométhane et on lave avec 40ml d'acide chlorhydrique 1N et deux fois 40ml d'eau. On sèche sur sulfate de magnésium et on évapore sous vide.

Le concentrat est purifié par chromatographie sur colonne de silice en utilisant comme éluant un mélange ternaire n-butanol / acétate d'éthyle / cyclohexane 30/20/30. Sur les 65 fractions de 10ml recueillies, on concentre les fractions 51 à 65.

Rendement : 1,52g (48%)

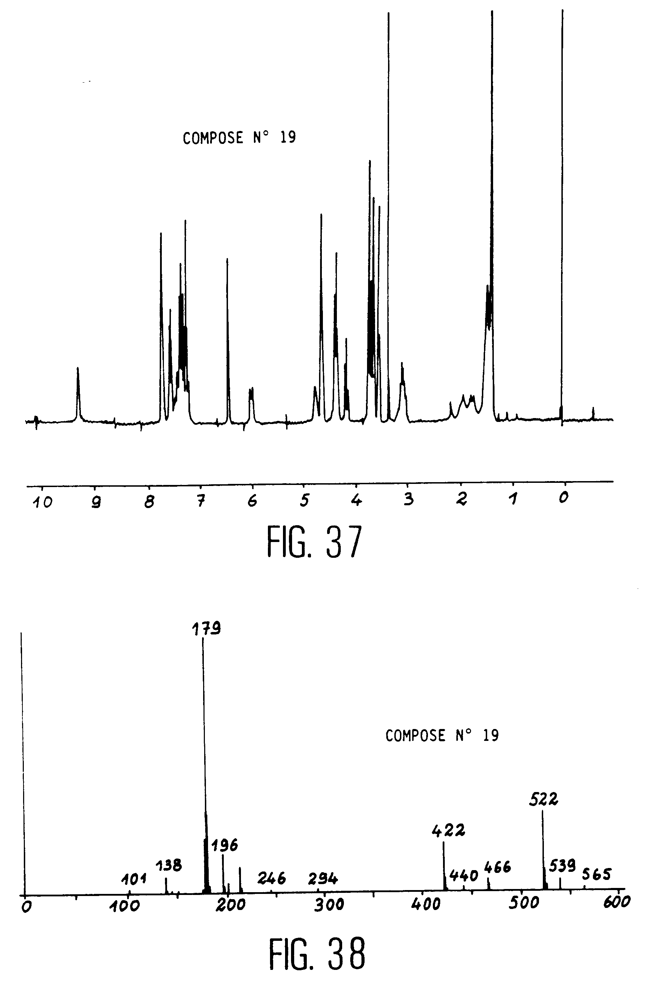

Les caractéristiques du produit sont les suivantes :
1°) F = 147° - 147,5°C
2°) C.C.M.
   Silice fluorescente
   K6F ; solvant d'élution : n-butanol / acétate d'éthyle / cyclohexane 30/20/30.
   Révélation : à 254nm et 356nm (spot bleu violet)
   Rf : 0,63.
3°) Spectre RMN : la figure 37 représente le spectre du proton qui confirme bien la structure du produit obtenu.
4°) Spectre de masse : (ionisation chimique à l'ammoniac). La figure 38 représente le spectre obtenu. Les pics à 522 et 539 (522 + 17) confirment bien la masse du produit (743) qui subit une fragmentation au niveau du groupe FMOC (743-222+1).
   Dans les exemples donnés ci-dessus, les composés n° 4, 10, 12 à 17 et 19 sont utilisables comme intermédiaires pour la préparation de substrats d'enzymes, en particulier polypeptidiques dans le cas des composés n° 12 à 17 et 19.
   Les composés n° 13, 14, 16 et 18 sont utilisables comme substrats d'enzymes.

## Revendications (Revendications pour l'(les) Etat(s) contractant(s) suivant(s): AT, BE, CH, DE, DK, FR, GB, GR, IT, LI, LU, MC, NL, PT, SE)

1. Dérivé de coumarine répondant à la formule : 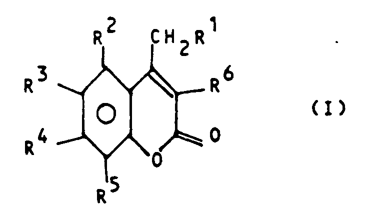 dans laquelle
1) R¹ représente un radical répondant aux formules
- (OCH₂CH₂)ₘOCH₂R⁷, ou
- (OCH(CH₃)CH₂)ₘOCH₂R⁷,
dans lesquelles m est un nombre entier de 1 à 30, et R⁷ représente
- un atome d'hydrogène,
- un atome d'halogène,
- OH,
- OR⁸,
- SH,
- SR⁸,
- COOH,
- COOR⁹,
- CONHR⁸, CONR⁸R¹⁰, CONHNH₂, 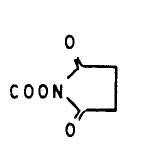
- NH₂,
- NHR⁸,
- NR⁸R¹⁰,
- SO₃H,
- SO₃R⁹, ou
- un radical alkyle, alcoxy ou aryle, non substitué ou substitué par au moins un substituant choisi parmi les atomes d'halogène, le radical phényle, CF₃, OH, OR⁸, SH, SR⁸, COOH, COOR⁹, NH₂, NHR⁸, NR⁸R¹⁰, SO₃H et SO₃R⁹,
où R⁸ est un radical alkyle ou aryle, R⁹ est un radical alkyle ou aryle, NH₄ ou M_{1/v} avec M étant un métal de valence v, et R¹⁰ est un radical alkyle ou aryle, ou R⁸ et R¹⁰ peuvent former ensemble un cycle hydrocarboné, saturé ou insaturé comportant éventuellement un hétéroatome choisi parmi O, S et N,
2) R², R³, R⁴ et R⁵ qui peuvent être identiques ou différents, représentent
- un atome d'hydrogène,
- un atome d'halogène,
- NH₂,
- NHR⁸,
- NR⁸R¹⁰,
- NHR¹¹,
- NR⁸R¹¹,
- SH
- SR⁸
- SR¹¹
- OH
- OR⁸
- OR¹¹
avec R⁸ et R¹⁰ ayant la signification donnée en 1) ci-dessus, et R¹¹ représentant un radical acyle dérivé d'un composé choisi parmi les acides aminés protégés ou non protégés, les peptides protégés ou non protégés, les acides carboxyliques et les acides gras, le radical PO₃R₂ avec R étant un atome d'hydrogène ou un radical alkyle, ou un radical dérivé d'un sucre, ou
- un radical alkyle ou alcoxy, non substitué ou substitué par au moins un substituant choisi parmi les atomes d'halogène, OH, OR⁸, SH, SR⁸, COOH, COOR⁹, NH₂, NHR⁸, NR⁸R¹⁰, SO₃H, SO₃R⁹, SR¹¹, OR¹¹, CF₃, NHR¹¹ et NR⁸R¹¹ avec R⁸, R⁹, R¹⁰ et R¹¹ ayant la signification donnée en 1) ci-dessus, ou
3) R² et R³, R³ et R⁴ ou R⁴ et R⁵ forment ensemble un noyau cyclique saturé ou insaturé de 5 à 8 atomes de carbone, ou un noyau hétérocyclique comportant au moins un hétéroatome choisi parmi N, O et S, ou
4) R³, R⁴ et R⁵ forment avec le noyau phényle auquel ils sont liés un noyau polycyclique, à trois cycles condensés comportant éventuellement un ou plusieurs hétéroatomes choisis parmi N, O et S, ou
5) R², R³ et R⁴ forment avec le noyau phényle auquel ils sont liés un noyau polycyclique, à trois cycles condensés, comportant éventuellement un ou plusieurs hétéroatomes choisis parmi N, O et S, et
6) R⁶ représente
- un atome d'hydrogène,
- un atome d'halogène,
- COOH,
- COOR⁹ avec R⁹ ayant la signification donnée ci-dessus, ou
- un radical alkyle, non substitué ou substitué par au moins un substituant choisi parmi les atomes d'halogène/ OH, SH, COOH, COOR⁹, OR⁸, SR⁸, SO₃H et SO₃R⁹ avec R⁸ et R⁹ ayant la signification donnée en 1) ci-dessus.

2. Dérivé de coumarine selon la revendication 1, caractérisé en ce que au moins l'un des R², R³, R⁴ et R⁵ représente NH₂, NHR¹¹, NHR⁸, NR⁸R¹¹, SH, OH, SR¹¹ ou OR¹¹ avec R⁸ et R¹¹ ayant la signification donnée dans la revendication 1.

3. Dérivé de coumarine selon la revendication 2, caractérisé en ce que R⁴ représente NH₂, NHR⁸, NHR¹¹, SH ou OH, et en ce que R², R³ et R⁵ représentent un atome d'hydrogène.

4. Dérivé selon la revendication 3, caractérisé en ce que R⁴ représente NH₂, OH ou un radical choisi parmi les radicaux de formule :

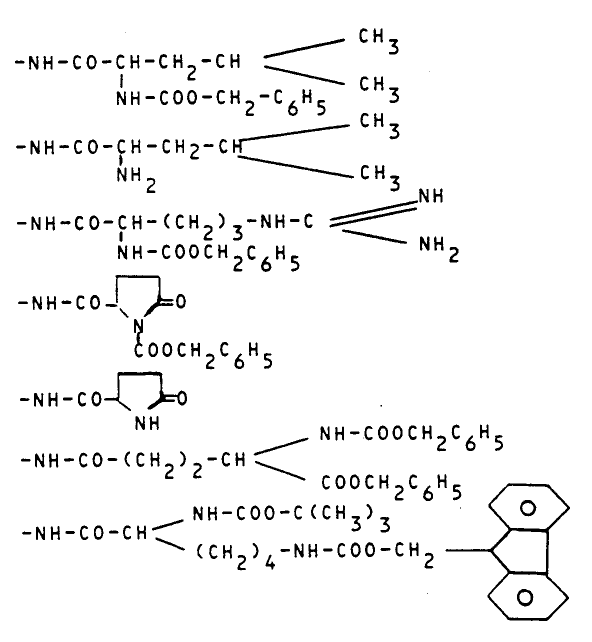

5. Dérivé selon la revendication 2, caractérisé en ce que R⁴ représente NHR¹¹, NR⁸R¹¹, SR¹¹ ou OR¹¹.

6. Dérivé selon la revendication 5, caractérisé en ce que R⁴ représente NHR¹¹ avec R¹¹ étant un radical choisi parmi les radicaux de formule :

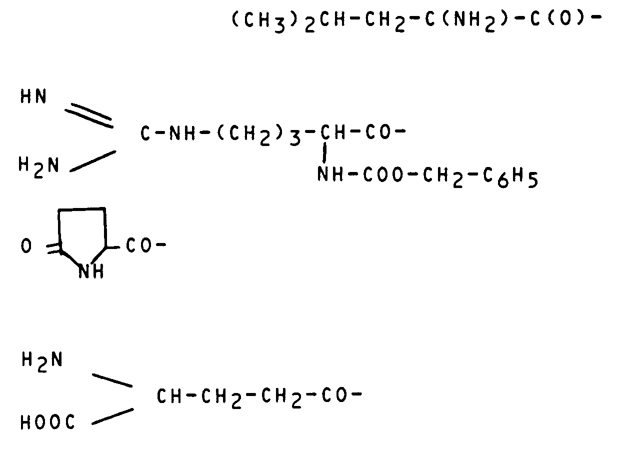

7. Dérivé selon la revendication 6, caractérisé en ce que R¹ représente (OCH₂CH₂)₂ OCH₃ et R², R³, R⁵ et R⁶ représentent un atome d'hydrogène.

8. Dérivé selon la revendication 4, caractérisé en ce que R¹ représente (OCH₂CH₂)₂OCH₃ et R², R³, R⁵ et R⁶ représentent un atome d'hydrogène.

9. Dérivé de coumarine selon l'une quelconque des revendications 1 à 3 et 5, caractérisé en ce que R⁶ représente un atome d'hydrogène.

10. Dérivé de coumarine selon l'une quelconque des revendications 2,3 et 5, caractérisé en ce que R¹ représente (OCH₂CH₂)ₘOCH₃.

11. Dérivé de coumarine selon revendication 10 caractérisé en ce que m est égal à 2.

12. Dérivé de coumarine selon la revendication 1, caractérisé en ce que au moins l'un des R², R³, R⁴ et R⁵ représente NH₂, NHR⁸, NR⁸R¹⁰, OH, OR⁸, SH, SR⁸ ou un radical alkyle substitué ou non substitué.

13. Dérivé de coumarine selon la revendication 1, caractérisé en ce que, soit R², R³ et R⁴, soit R³, R⁴ et R⁵ forment avec le noyau phényle auquel ils sont liés un noyau polycyclique comportant trois cycles condensés et un atome d'azote.

14. Ester β-cétonique répondant à la formule : 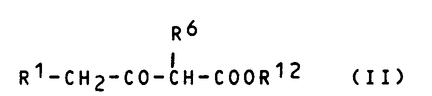 dans laquelle R¹ et R⁶ ont la signification donnée dans la revendication 1 et R¹² est un radical alkyle, aryle ou cycloalkyle.

15. Ester β-cétonique selon la revendication 14, caractérisé en ce que R⁶ est un atome d'hydrogène.

16. Ester β-cétonique selon l'une quelconque des revendications 14 et 15, caractérisé en ce que R¹² est un radical alkyle de 1 à 4 atomes de carbone.

17. Ester β-cétonique selon la revendication 16, caractérisé en ce que R¹² est le radical éthyle.

18. Ester β-cétonique selon l'une quelconque des revendications 14 à 17, caractérisé en ce que R¹ représente le radical de formule
-(OCH₂CH₂)ₘOCH₂R⁷
dans laquelle R⁷ est un atome d'hydrogène ou COOR⁹ avec R⁹ étant le radical benzyle.

19. Procédé de préparation d'un ester β-cétonique de formule (II) selon l'une quelconque des revendications 14 à 18, caractérisé en ce qu'il comprend les étapes suivantes :
1°) transformer un acide de formule R¹-CH₂-COOH dans laquelle R¹ a la signification donnée dans la revendication 1, en l'imidazolide ou le chlorure d'acide correspondant,
2°) faire réagir l'imidazolide ou le chlorure d'acide avec un ester cyclique de formule : 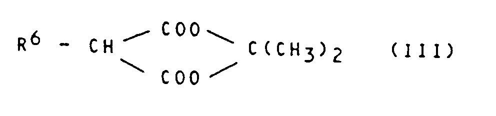 pour former un dérivé acylé, et
3°) faire réagir le dérivé acylé avec un alcool de formule R¹²OH pour obtenir l'ester β-cétonique de formule (II).

20. Procédé de préparation d'un dérivé de coumarine de formule (I), selon l'une quelconque des revendications 1 à 13, caractérisé en ce qu'il consiste à faire réagir un ester β-cétonique de formule : 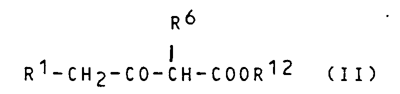 avec un phénol de formule : 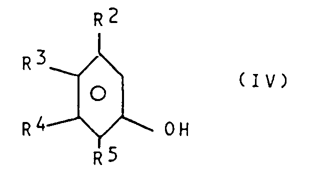 dans lesquelles R¹, R², R³, R⁴, R⁵ et R^{6,} ont les significations données dans le revendication 1 et R¹² représente un radical alkyle, aryle ou cycloalkyle.

21. Utilisation des dérivés de coumarine selon l'une quelconque des revendications 3, 4 et 8 pour la synthèse d'un substrat d'enzyme destiné au dosage de cette enzyme.

22. Utilisation des dérivés de coumarine selon l'une quelconque des revendications 5, 6 et 7 comme substrat d'enzyme dans un procédé de dosage de cette enzyme.

## Revendications (Revendications pour l'(les) Etat(s) contractant(s) suivant(s): ES)

1. Procédé de préparation d'un dérivé de coumarine répondant à la formule : 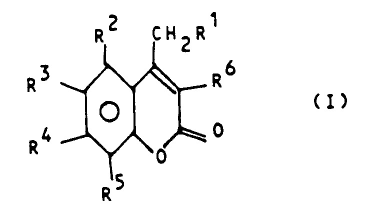 dans laquelle
1) R¹ représente un radical répondant aux formules
- (OCH₂CH₂)ₘOCH₂R⁷, ou
- (OCH(CH₃)CH₂)ₘOCH₂R⁷,
dans lesquelles m est un nombre entier de 1 à 30, et R⁷ représente
- un atome d'hydrogène,
- un atome d'halogène,
- OH,
- OR⁸,
- SH,
- SR⁸,
- COOH,
- COOR⁹,
- CONHR⁸, CONR⁸R¹⁰, CONHNH₂, 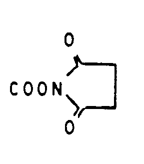
- NH₂,
- NHR⁸,
- NR⁸R¹⁰,
- SO₃H,
- SO₃R⁹, ou
- un radical alkyle, alcoxy ou aryle, non substitué ou substitué par au moins un substituant choisi parmi les atomes d'halogène, le radical phényle, CF₃, OH, OR⁸, SH, SR⁸, COOH, COOR⁹, NH₂, NHR⁸, NR⁸R¹⁰, SO₃H et SO₃R⁹,
où R⁸ est un radical alkyle ou aryle, R⁹ est un radical alkyle ou aryle, NH₄ ou M_{1/v} avec M étant un métal de valence v, et R¹⁰ est un radical alkyle ou aryle, ou R⁸ et R¹⁰ peuvent former ensemble un cycle hydrocarboné, saturé ou insaturé comportant éventuellement un hétéroatome choisi parmi O, S et N,
2) R², R³, R⁴ et R⁵ qui peuvent être identiques ou différents, représentent
- un atome d'hydrogéne,
- un atome d'halogène,
- NH₂,
- NHR⁸,
- NR⁸R¹⁰,
- NHR¹¹,
- NR⁸R¹¹,
- SH
- SR⁸
- SR¹¹
- OH
- OR⁸
- OR¹¹
avec R⁸ et R¹⁰ ayant la signification donnée en 1) ci-dessus, et R¹¹ représentant un radical acyle dérivé d'un composé choisi parmi les acides aminés protégés ou non protégés, les peptides protégés ou non protégés, les acides carboxyliques et les acides gras, le radical PO₃R₂ avec R étant un atome d'hydrogène ou un radical alkyle, ou un radical dérivé d'un sucre, ou
- un radical alkyle ou alcoxy, non substitué ou substitué par au moins un substituant choisi parmi les atomes d'halogène, OH, OR⁸, SH, SR⁸, COOH, COOR⁹, NH₂, NHR⁸, NR⁸R¹⁰, SO₃H, SO₃R⁹, SR¹¹, OR¹¹, CF₃, NHR¹¹ et NR⁸R¹¹ avec R⁸, R⁹, R¹⁰ et R¹¹ ayant la signification donnée en 1) ci-dessus, ou
3) R² et R³, R³ et R⁴ ou R⁴ et R⁵ forment ensemble un noyau cyclique saturé ou insaturé de 5 à 8 atomes de carbone, ou un noyau hétérocyclique comportant au moins un hétéroatome choisi parmi N, O et S, ou
4) R³, R⁴ et R⁵ forment avec le noyau phényle auquel ils sont liés un noyau polycyclique, à trois cycles condensés comportant éventuellement un ou plusieurs hétéroatomes choisis parmi N, O et S, ou
5) R², R³ et R⁴ forment avec le noyau phényle auquel ils sont liés un noyau polycyclique, à trois cycles condensés, comportant éventuellement un ou plusieurs hétéroatomes choisis parmi N, O et S, et
6) R⁶ représente
- un atome d'hydrogène,
- un atome d'halogène,
- COOH,
- COOR⁹ avec R⁹ ayant la signification donnée ci-dessus, ou
- un radical alkyle, non substitué ou substitué par au moins un substituant choisi parmi les atomes d'halogène, OH, SH, COOH, COOR⁹, OR⁸, SR⁸, SO₃H et SO₃R⁹ avec R⁸ et R⁹ ayant la signification donnée en 1) ci-dessus,
caractérisé en ce qu'il consiste à faire réagir un ester β-cétonique de formule : 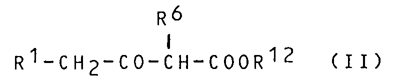 avec un phénol de formule : 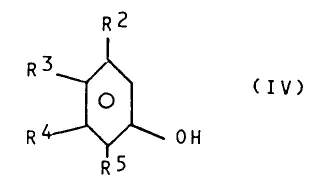 dans lesquelles R¹, R², R³, R⁴, R⁵ et R⁶ ont les significations données dans les paragraphes 1) à 6) ci-dessus et R¹² représente un radical alkyle, aryle ou cycloalkyle.

2. Procédé selon la revendication 1, caractérisé en ce que au moins l'un des R², R³, R⁴ et R⁵ représente NH₂, NHR¹¹, NHR⁸, NR⁸R¹¹, SH, OH, SR¹¹ ou OR¹¹ avec R⁸ et R¹¹ ayant la signification donnée dans la revendication 1.

3. Procédé selon la revendication 2, caractérisé en ce que R⁴ représente NH₂, NHR⁸, NHR¹¹, SH ou OH, et en ce que R², R³ et R⁵ représentent un atome d' hydrogène.

4. Procédé selon la revendication 3, caractérisé en ce que R⁴ représente NH₂, OH ou un radical choisi parmi les radicaux de formule :

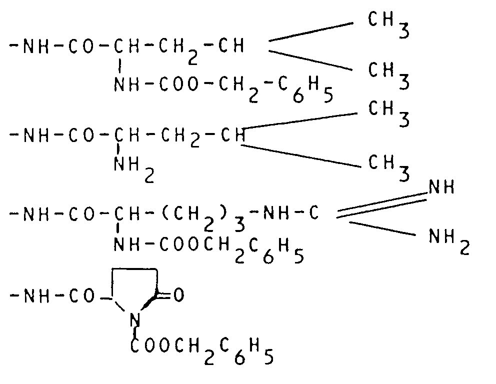

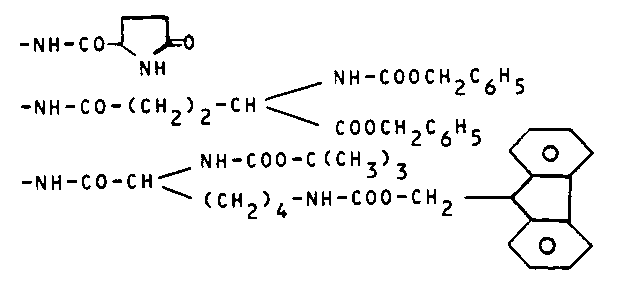

5. Procédé selon la revendication 2, caractérisé en ce que R⁴ représente NHR¹¹, NR⁸R¹¹, SR¹¹ ou OR¹¹.

6. Procédé selon la revendication 5, caractérisé en ce que R⁴ représente NHR¹¹ avec R¹¹ étant un radical choisi parmi les radicaux de formule :

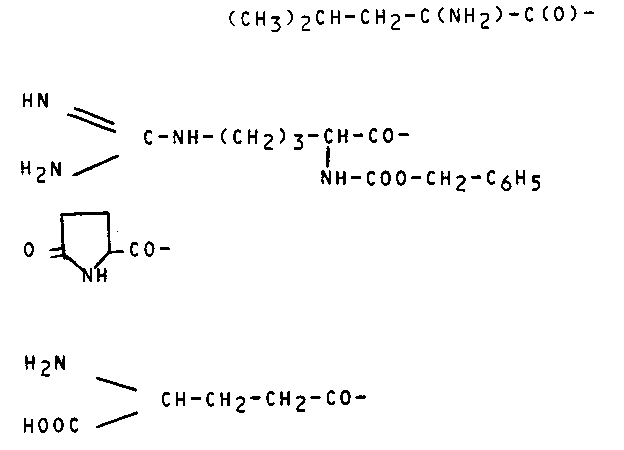

7. Procédé selon la revendication 6, caractérisé en ce que R¹ représente (OCH₂CH₂)₂OCH₃ et R², R³, R⁵ et R⁶ représentent un atome d'hydrogène.

8. Procédé selon la revendication 4, caractérisé en ce que R¹ représente (OCH₂CH₂)₂OCH₃ et R², R³, R⁵ et R⁶ représentent un atome d'hydrogène.

9. Procédé de coumarine selon l'une quelconque des revendications 1, 3 et 5, caractérisé en ce que R⁶ représente un atome d'hydrogène.

10. Procédé selon l'une quelconque des revendications 2, 3 et 5, caractérisé en ce que R¹ représente (OCH₂CH₂)ₘOCH₃.

11. Procédé selon la revendication 10, caractérisé en ce que m est égal à 2.

12. Procédé selon la revendication 1, caractérisé en ce que au moins l'un des R², R³, R⁴ et R⁵ représente NH₂, NHR⁸, NR⁸R¹⁰, OH, OR⁸, SH, SR⁸ ou un radical alkyle substitué ou non substitué.

13. Procédé selon la revendication 1, caractérisé en ce que, soit R², R³ et R⁴, soit R³, R⁴ et R⁵ forment avec le noyau phényle auquel ils sont liés un noyau polycyclique comportant trois cycles condensés et un atome d'azote.

14. Procédé de préparation d'un ester β-cétonique répondant à la formule : 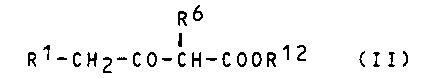 dans laquelle R¹ et R⁶ ont la signification donnée dans la revendication 1 et R¹² est un radical alkyle aryle ou cycloalkyle, caractérisé en ce qu'il comprend les étapes suivantes :
1°) transformer un acide de formule R¹-CH₂-COOH dans laquelle R¹ a la signification donnée dans la revendication 1, en l'imidazolide ou le chlorure d'acide correspondant,
2°) faire réagir l'imidazolide ou le chlorure d'acide avec un ester cyclique de formule : 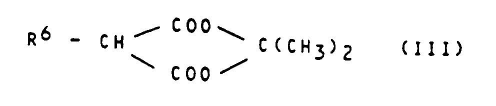 pour former un dérivé acylé, et
3°) faire réagir le dérivé acylé avec un alcool de formule R¹²OH pour obtenir l'ester β-cétonique de formule (II).

15. Procédé selon la revendication 14, caractérisé en ce que R⁶ est un atome d'hydrogène.

16. Procédé selon l'une quelconque des revendications 14 et 15, caractérisé en ce que R¹² est un radical alkyle de 1 à 4 atomes de carbone.

17. Procédé selon la revendication 16, caractérisé en ce que R¹² est un radical éthyle.

18. Procédé selon l'une quelconque des revendications 14 à 17, caractérisé en ce que R¹ représente le radical de formule
-(OCH₂CH₂)ₘOCH₂R⁷
dans laquelle R⁷ est un atome d'hydrogène ou COOR⁹ avec R⁹ étant le radical benzyle.

19. Utilisation des dérivés de coumarine obtenus par le procédé selon l'une quelconque des revendications 3, 4 et 8 pour la synthèse d'un substrat d'enzyme destiné au dosage de cette enzyme.

20. Utilisation des dérivés de coumarine obtenus par le procédé selon l'une quelconque des revendications 5, 6 et 7 comme substrat d'enzyme dans un procédé de dosage de cette enzyme.

## Claims (Claims for the following Contracting State(s): AT, BE, CH, DE, DK, FR, GB, GR, IT, LI, LU, MC, NL, PT, SE)

1. Coumarin derivative complying with the formula: 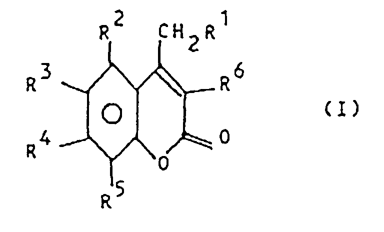 in which
1) R¹ represents a radical complying with the formulas
- (OCH₂CH₂)ₘOCH₂R⁷, or
- (OCH(CH₃)CH₂)ₘOCH₂R⁷,
in which m is an integer from 1 to 30 and R⁷ represents a hydrogen atom, a halogen atom, OH, OR⁸, SH, SR⁸, COOH, COOR⁹, CONHR⁸, CON⁸R¹⁰, CONHNH₂, 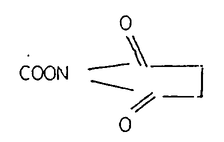 NH₂, NHR⁸, NR⁸R¹⁰, SO₃H, SO₃R⁹, or
- an alkyl, alkoxy or aryl radical, which is unsubstituted or substituted by at least one substituent chosen from among the halogen atoms, phenyl radical, CF₃, OH, OR⁸, SH, SR⁸, COOH, COOR⁹, NH₂, NHR⁸, NR⁸R¹⁰, SO₃H and SO₃R⁹, in which R⁸ is alkyl or aryl radical, R⁹ an alkyl or aryl radical, NH₄ or M_{1 /v} with M being a valency metal v and R¹⁰ is an alkyl or aryl radical or R⁸ and R¹⁰ can together form a saturated or unsaturated hydrocarbon cycle optionally incorporating a heteroatom chosen from among O, S and N,
2) R², R³, R⁴ and R⁵, which can be the same or different, represent a hydrogen atom, a halogen atom, NH₂, NHR⁸, NR⁸R¹⁰, NHR¹¹, NR⁸R¹¹ , SH, SR⁸, SR¹¹ , OH, OR⁸, OR¹¹ with R⁸ and R¹⁰ having the meaning given in 1) hereinbefore and R¹¹ representing an acyl radical derived from a compound chosen from among protected or unprotected amino acids, protected or unprotected peptides. carboxylic acids and fatty acids, the radical PO₃R₂ with R being a hydrogen atom or an alkyl radical, or a radical derived from a sugar, or an alkoxy or alkyl radical, which is unsubstituted or substituted by at least one substituent chosen from among the halogen atoms, OH, OR⁸, SH, SR⁸, COOH, COOR⁹, NH₂, NHR⁸, NR⁸R¹⁰, SO₃H, SO₃R⁹, SR¹¹, OR¹¹, CF₃, NHR¹¹ and NR⁸R¹¹ with R⁸, R⁹, R¹⁰ and R¹¹ having the meanings given in 1) hereinbefore, or
3) R² and R³, R³ and R⁴ or R⁴ and R⁵ together form a saturated or unsaturated cyclic nucleus with 5 to 8 carbon atoms, or a heterocyclic nucleus having at least one heteroatom chosen from among N, O and S, or
4) R³, R⁴ and R⁵ form together with the phenyl nucleus to which they are linked a polycyclic nucleus having three condensed cycles and optionally having one or more heteroatoms chosen from among N, O and S, or
5) R², R³ and R⁴ form with the phenyl nucleus to which they are linked a polycyclic nucleus having three condensed cycles and optionally one or more heteroatoms chosen from among N, O and S and
6) R⁶ represents a hydrogen atom, a halogen atom, COOH, COOR⁹ with R⁹ having the meaning given hereinbefore, or an alkyl radical, which is unsubstituted or substituted by at least one substituent chosen from among the halogen atoms, OH, SH, COOH, COOR⁹, OR⁸, SR⁸, SO₃H and SO₃R⁹ with R⁸ and R⁹ having the meaning given in 1) hereinbefore.

2. Coumarin derivative according to claim 1, characterized in that at least one of the R²., R³, R⁴ and R⁵ represents NH₂, NHR¹¹ , NHR⁸, NR⁸R¹¹, SH, OH, SR¹¹ or OR¹¹ with R⁸ and R¹¹ having the meanings given in claim 1.

3. Coumarin derivative according to claim 2, characterized in that R⁴ represents NH₂, NHR⁸, NHR¹¹ , SH or OH and in that R², R³ and R⁵ represent a hydrogen atom.

4. Derivative according to claim 3, characterized in that R⁴ represents NH₂, OH or a radical chosen from among those of formula:

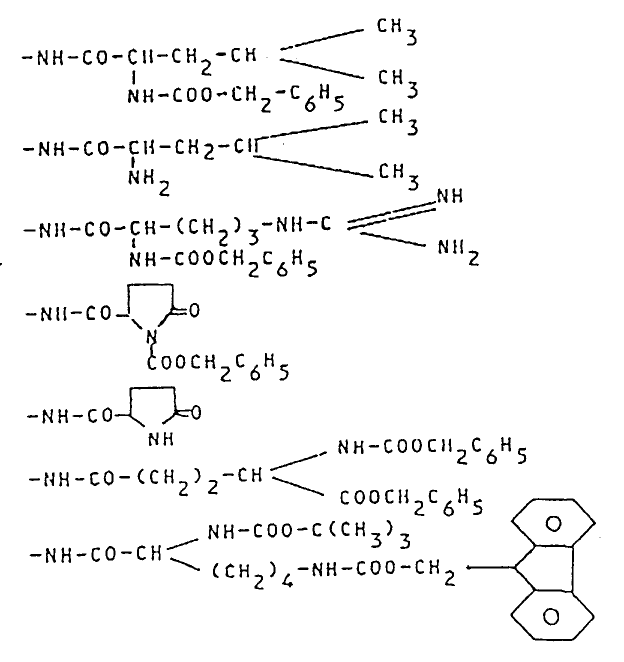

5. Derivative according to claim 2, characterized in that R⁴ represents NHR¹¹, NR⁸R¹¹, SR¹¹ or OR¹¹.

6. Derivative according to claim 5, characterized in that R⁴ represents NHR¹¹ with R¹¹ being a radical chosen from among those of formula:

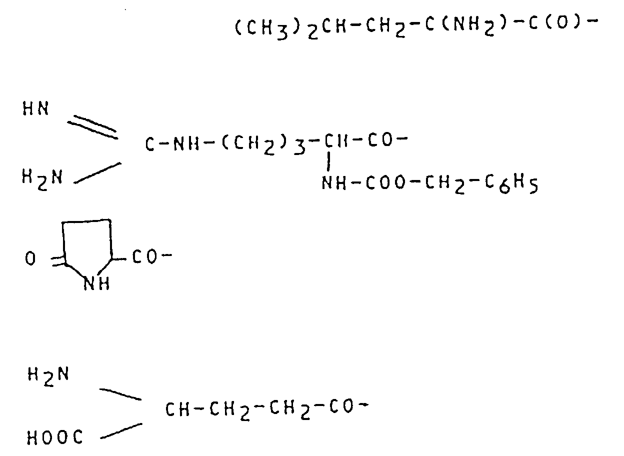

7. Derivative according to claim 6, characterized in that R¹ represents (OCH₂CH₂)₂OCH₃ and R², R³, R⁵ and R⁶ represent a hydrogen atom.

8. Derivative according to claim 4, characterized in that R¹ represents (OCH₂CH₂)₂OCH₃ and R², R³, R⁵ and R⁶ represent a hydrogen atom.

9. Coumarin derivative according to any one of the claims 1 to 3 and 5, characterized in that R⁶ represents a hydrogen atom.

10. Coumarin derivative according to any one of the claims 2, 3 and 5, characterized in that R¹ represents (OCH₂CH₂)ₘOCH₃.

11. Coumarin derivative according to claim 10, characterized in that m is equal to 2.

12. Coumarin derivative according to claim 1, characterized in that at least one of the R², R³, R⁴ and R⁵ represents NH₂, NHR⁸, NR⁸R¹⁰, OH, OR⁸, SH, SR⁸ or an alkyl radical in substituted or unsubstituted form.

13. Coumarin derivative according to claim 1, characterized in that either R², R³ and R⁴ or R³, R⁴ and R⁵ form together with the phenyl nucleus to which they are connected a polycyclic nucleus having three condensed cycles and a nitrogen atom.

14. β-ketonic ester according to formula: 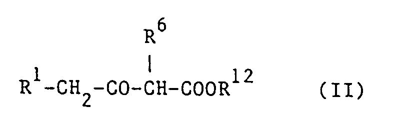 in which R¹ and R⁶ have the meanings given in claim 1 and R¹² is an alkyl, aryl or cycloalkyl radical.

15. β-ketonic ester according to claim 14, characterized in that R⁶ is a hydrogen atom.

16. β-ketonic ester according to either of the claims 14 and 15, characterized in that R¹² is an alkyl radical with 1 to 4 carbon atoms.

17. β-ketonic ester according to claim 16, characterized in that R¹² is the ethyl radical.

18. β-ketonic ester according to any one of the claims 14 to 17, characterized in that R¹ represents the radical of formula:
-(OCH₂CH₂)ₘOCH₂R⁷
in which R⁷ is a hydrogen atom or COOR⁹ with R⁹ being the benzyl radical.

19. Process for the preparation of a β-ketonic ester of formula (II) according to any one of the claims 14 to 18, characterized in that it comprises the following stages:
1°) transforming an acid of formula R¹-CH₂-COOH, in which R¹ has the meaning given in claim 1, into the corresponding acid chloride or imidazolide,
2°) reacting the acid chloride or imidazolide with a cyclic ester of formula: 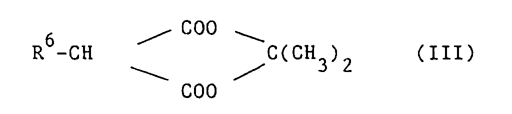 to form an acylated derivative and
3°) reacting the acylated derivative with an alcohol of formula R¹²OH for obtaining the β-ketonic ester of formula (II).

20. Process for the preparation of a coumarin derivative of formula (I), according to any one of the claims 1 to 13, characterized in that it consists of reacting a β-ketonic ester of formula: 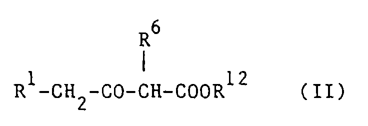 with a phenol of formula: 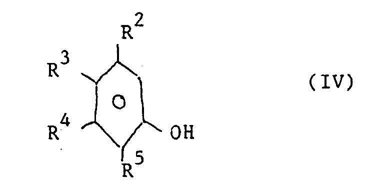 in which R¹, R², R³, R⁴ and R⁵ have the meanings given in claim 1 and R¹² represents an alkyl, aryl or cyclic alkyl radical.

21. Use of coumarin derivatives according to any one of the claims 3, 4 and 8, for the synthesis of an enzyme substrate for the dosing of said enzyme.

22. Use of coumarin derivatives according to any one of the claims 5, 6 and 7, as an enzyme substrate in a process for dosing said enzyme.

## Claims (Claims for the following Contracting State(s): ES)

1. Process for the preparation of a coumarin derivative complying with the formula: 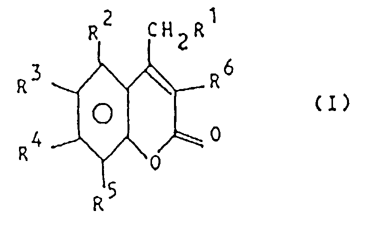 in which
1) R¹ represents a radical complying with the formulas
- (OCH₂CH₂)ₘOCH₂R⁷, or
- (OCH(CH₃)CH₂)ₘOCH₂R⁷,
in which m is an integer from 1 to 30 and R⁷ represents a hydrogen atom, a halogen atom, OH, OR⁸, SH, SR⁸, COOH, COOR⁹, CONHR⁸, CONR⁸R¹⁰, CONHNH₂, 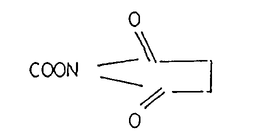 NH₂, NHR⁸, NR⁸R¹⁰, SO₃H, SO₃R⁹, or
- an alkyl, alkoxy or aryl radical, which is unsubstituted or substituted by at least one substituent chosen from among the halogen atoms, phenyl radical, CF₃, OH, OR⁸, SH, SR⁸, COOH, COOR⁹, NH₂, NHR⁸, NR⁸R¹⁰, SO₃H and SO₃R⁹, in which R⁸ is alkyl or aryl radical, R⁹ an alkyl or aryl radical, NH₄ or M_{1/v} with M being a valency metal v and R¹⁰ is an alkyl or aryl radical or R⁸ and R¹⁰ can together form a saturated or unsaturated hydrocarbon cycle optionally incorporating a heteroatom chosen from among O, S and N,
2) R², R³, R⁴ and R⁵, which can be the same or different, represent a hydrogen atom, a halogen atom, NH₂, NHR⁸, NR⁸R¹⁰, NHR¹¹, NR⁸R¹¹, SH, SR⁸, SR¹¹, OH, OR⁸, OR¹¹ with R⁸ and R¹⁰ having the meaning given in 1) hereinbefore and R¹¹ representing an acyl radical derived from a compound chosen from among protected or unprotected amino acids, protected or unprotected peptides, carboxylic acids and fatty acids, the radical PO₃R₂ with R being a hydrogen atom or an alkyl radical, or a radical derived from a sugar, or an alkoxy or alkyl radical, which is unsubstituted or substituted by at least one substituent chosen from among the halogen atoms OH, OR⁸, SH, SR⁸, COOH, COOR⁹, NH₂, NHR⁸, NR⁸R¹⁰, SO₃H, SO₃R⁹ SR¹¹, OR¹¹, CF₃, NHR¹¹ and NR⁸R¹¹ with R⁸, R⁹, R¹⁰ and R¹¹ having the meanings given in 1) hereinbefore, or
3) R² and R³, R³ and R⁴ or R⁴ and R⁵ together form a saturated or unsaturated cyclic nucleus with 5 to 8 carbon atoms, or a heterocyclic nucleus having at least one heteroatom chosen from among N, O and S, or
4) R³, R⁴ and R⁵ form together with the phenyl nucleus to which they are linked a polycyclic nucleus having three condensed cycles and optionally having one or more heteroatoms chosen from among N, O and S, or
5) R², R³ and R⁴ form with the phenyl nucleus to which they are linked a polycyclic nucleus having three condensed cycles and optionally one or more heteroatoms chosen from among N, O and S, and
6) R⁶ represents a hydrogen atom, a halogen atom, COOH, COOR⁹ with R⁹ having the meaning given hereinbefore, or an alkyl radical, which is unsubstituted or substituted by at least one substituent chosen from among the halogen atoms, OH, SH, COOH, COOR⁹, OR⁸, SR⁸, SO₃H and SO₃R⁹ with R⁸ and R⁹ having the meaning given in 1) hereinbefore,
characterized in that it consists of reacting a β-ketonic acid of formula: 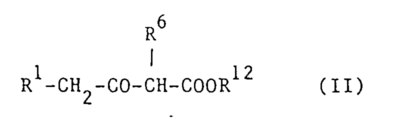 with a phenol of formula: 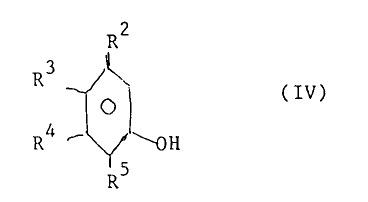 in which R¹, R², R³, R⁴, R⁵ and R⁶ have the meanings given in the above paragraphs 1) to 6) and R¹² represents an alkyl aryl or cycloalkyl radical.

2. Process according to claim 1, characterized in that at least one of the R², R³, R⁴ and R⁵ represents NH₂, NHR¹¹, NHR⁸, NR⁸R¹¹, SH, OH, SR¹¹ or OR¹¹ with R⁸ and R¹¹ having the meanings given in claim 1.

3. Process according to claim 2, characterized in that R⁴ represents NH₂, NHR⁸, NHR¹¹, SH or OH and in that R², R³ and R⁵ represent a hydrogen atom.

4. Process according to claim 3, characterized in that R⁴ represents NH₂, OH or a radical chosen from among those of formula:

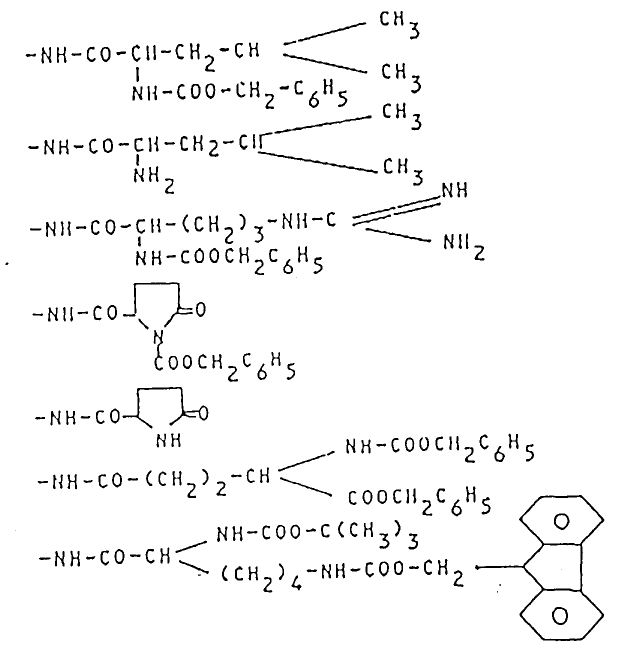

5. Process according to claim 2, characterized in that R⁴ represents NHR¹¹, NR⁸R¹¹, SR¹¹ or OR¹¹.

6. Process according to claim 5, characterized in that R⁴ represents NHR¹¹ with R¹¹ being a radical chosen from among those of formula:

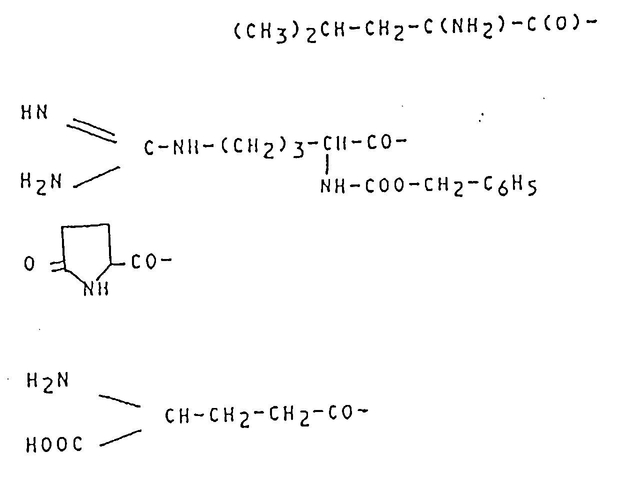

7. Process according to claim 6, characterized in that R¹ represents (OCH₂CH₂)₂OCH₃ and R², R³, R⁵ and R⁶ represent a hydrogen atom.

8. Process according to claim 4, characterized in that R¹ represents (OCH₂CH₂)₂OCH₃ and R², R³, R⁵ and R⁶ represent a hydrogen atom.

9. Process according to any one of the claims 1 to 3 and 5, characterized in that R⁶ represents a hydrogen atom.

10. Process according to any one of the claims 2, 3 and 5, characterized in that R¹ represents (OCH₂CH₂)ₘOCH₃.

11. Process according to claim 10, characterized in that m is equal to 2.

12. Process according to claim 1, characterized in that at least one of the R², R³, R⁴ and R⁵ represents NH₂, NHR⁸, NR⁸R¹⁰, OH, OR⁸, SH, SR⁸ or an alkyl radical in substituted or unsubstituted form.

13. Process according to claim 1, characterized in that either R², R³ and R⁴ or R³, R⁴ and R⁵ form together with the phenyl nucleus to which they are connected a polycyclic nucleus having three condensed cycles and a nitrogen atom.

14. Process for the preparation of a β-ketonic ester according to formula: 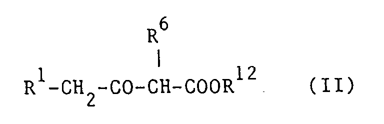 in which R¹ and R⁶ have the meanings given in claim 1 and R¹² is an alkyl, aryl or cycloalkyl radical, characterized in that it comprises the following steps:
1) transforming an acid of formula R¹-CH₂-COOH in which R¹ has the meaning given in claim 1, into the corresponding acid chloride or imidazolide,
2) reacting the acid chloride or imidazolide with a cyclic ester of formula: 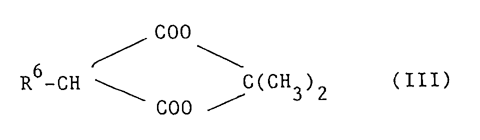 in order to form an acylated derivative and
3) reacting the acylated derivative with an alcohol of formula R¹²OH in order to obtain the β-ketonic ester of formula (II).

15. Process according to claim 14, characterized in that R⁶ is a hydrogen atom.

16. Process according to either of the claims 14 and 15, characterized in that R¹² is an alkyl radical with 1 to 4 carbon atoms.

17. Process according to claim 16, characterized in that R¹² is the ethyl radical.

18. Process according to any one of the claims 14 to 17, characterized in that R¹ represents the radical of formula:
-(OCH₂CH₂)ₘOCH₂R⁷
in which R⁷ is a hydrogen atom or COOR⁹ with R⁹ being the benzyl radical.

19. Use of coumarin derivatives according to any one of the claims 3, 4 and 8 for the synthesis of an enzyme substrate for the dosing of said enzyme.

20. Use of coumarin derivatives according to any one of the claims 5, 6 and 7 as an enzyme substrate in a process for dosing said enzyme.

## Patentansprüche (Patentansprüche für folgende(n) Vertragsstaat(en): AT, BE, CH, DE, DK, FR, GB, GR, IT, LI, LU, MC, NL, PT, SE)

1. Cumarinderivat entsprechend der Formel: 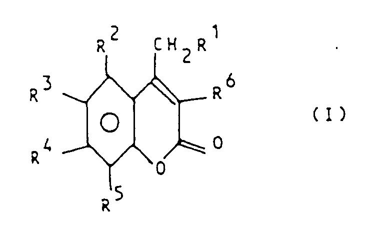 worin
1) R¹ einen Rest darstellt, der den Formeln
- (OCH₂CH₂)ₘOCH₂R⁷ oder
- (OCH(CH₃)CH₂)ₘOCH₂R⁷ entspricht,
worin m eine ganze Zahl von 1 bis 30 ist und R⁷
- ein Wasserstoffatom,
- ein Halogenatom,
- OH,
- OR⁸,
- SH,
- SR⁸,
- COOH,
- COOH⁹,
- CONHR⁸, CONR⁸R¹⁰, CONHNH₂, 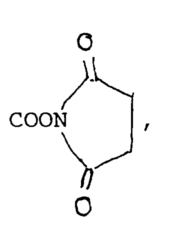
- NH₂,
- NHR⁸,
- NR⁸R¹⁰,
- SO₃H,
- SO₃R⁹ oder
- einen unsubstituierten oder mit mindestens einem Substituenten, der gewählt ist unter den Halogenatomen, dem Phenylrest, CF₃, OH, OR⁸, SH, SR⁸, COOH, COOR⁹, NH₂, NHR⁸, NR⁸R¹⁰, SO₃H und SO₃R⁹, substituierten Alkyl-, Alkoxy- oder Arylrest darstellt,
wo R⁸ ein Alkyl- oder Arylrest ist, R⁹ ein Alkyl- oder Arylrest, NH₄ oder M_{1/v} ist, wobei M ein Metall der Wertigkeit v ist, und R¹⁰ ein Alkyl- oder Arylrest ist oder wo R⁸ und R¹⁰ zusammen einen gesättigten oder ungesättigten Kohlenwasserstoffring bilden können, der gegebenenfalls ein unter O, S und N ausgewähltes Heteroatom enthalten kann,
2) R², R³, R⁴ und R⁵, die gleich oder verschieden sein können,
- ein Wasserstoffatom,
- ein Halogenatom,
- NH₂,
- NHR⁸,
- NR⁸R¹⁰,
- NHR¹¹,
- NR⁸R¹¹,
- SH,
- SR⁸,
- SR¹¹,
- OH,
- OR⁸,
- OR¹¹,
wo R⁸ und R¹⁰ die oben in 1) gegebene Bedeutung haben und R¹¹ einen Acylrest darstellt, der abgeleitet ist von einer Verbindung, die unter den geschützten oder nicht geschützten Aminosäuren, den geschützten oder nicht geschützten Peptiden, den Carbonsäuren und den Fettsäuren, dem Rest PO₃R₂, wobei R ein Wasserstoffatom oder ein Alkylrest ist, oder einem von einem Zucker abgeleiteten Rest gewählt ist, oder
- einen nicht substituierten oder mit mindestens einem Substituenten, der gewählt ist unter den Halogenatomen, OH, OR⁸, SH, SR⁸, COOH, COOR⁹, NH₂, NHR⁸, NR⁸R¹⁰, SO₃H, SO₃R⁹, SR¹¹, OR¹¹, CF₃, NHR¹¹ und NR⁸R¹¹, substituierten Alkyl- oder Alkoxyrest darstellen, wobei R⁸, R⁹, R¹⁰ und R¹¹ die oben in 1) gegebene Bedeutung haben, oder
3) R² und R³, R³ und R⁴ oder R⁴ und R⁵ zusammen einen ringförmigen gesättigten oder ungesättigten Kern von 5 bis 8 Kohlenstoffatomen oder einen heterocyclischen Kern bilden, der mindestens ein unter N, O und S gewähltes Heteroatom enthält, oder
4) R³, R⁴ und R⁵ mit dem Benzolkern, an den sie gebunden sind, einen polycyclischen Kern mit drei kondensierten Ringen bilden, der gegebenenfalls ein oder mehrere unter N, O und S gewählte Heteroatome enthält, oder
5) R², R³ und R⁴ mit dem Benzolkern, an den sie gebunden sind, einen polycyclischen Kern mit drei kondensierten Ringen bilden, der gegebenenfalls ein oder mehrere unter N, O und S gewählte Heteroatome enthält, und
6) R⁶
- ein Wasserstoffatom,
- ein Halogenatom,
- COOH,
- COOR⁹, wobei R⁹ die oben gegebene Bedeutung hat, oder
- einen nicht substituierten oder mit mindestens einem Substituenten, der gewählt ist unter den Halogenatomen, OH, SH, COOH, COOR⁹, OR⁸, SR⁸, SO₃H und SO₃R⁹, substituierten Alkylrest darstellt, wobei R⁸ und R⁹ die oben in 1) gegebene Bedeutung haben.

2. Cumarinderivat nach Anspruch 1, dadurch gekennzeichnet, daß mindestens einer der Substituenten R², R³, R⁴ und R⁵ für NH₂, NHR¹¹, NHR⁸, NR⁸R¹¹, SH, OH, SR¹¹ oder OR¹¹ steht, wobei R⁸ und R¹¹ die in Anspruch 1 gegebene Bedeutung haben.

3. Cumarinderivat nach Anspruch 2, dadurch gekennzeichnet, daß R⁴ für NH₂, NHR⁸, NHR¹¹, SH oder OH steht und daß R², R³ und R⁵ ein Wasserstoffatom darstellen.

4. Derivat nach Anspruch 3, dadurch gekennzeichnet, daß R⁴ für NH₂, OH oder einen Rest steht, der gewählt ist unter den Resten der Formel:

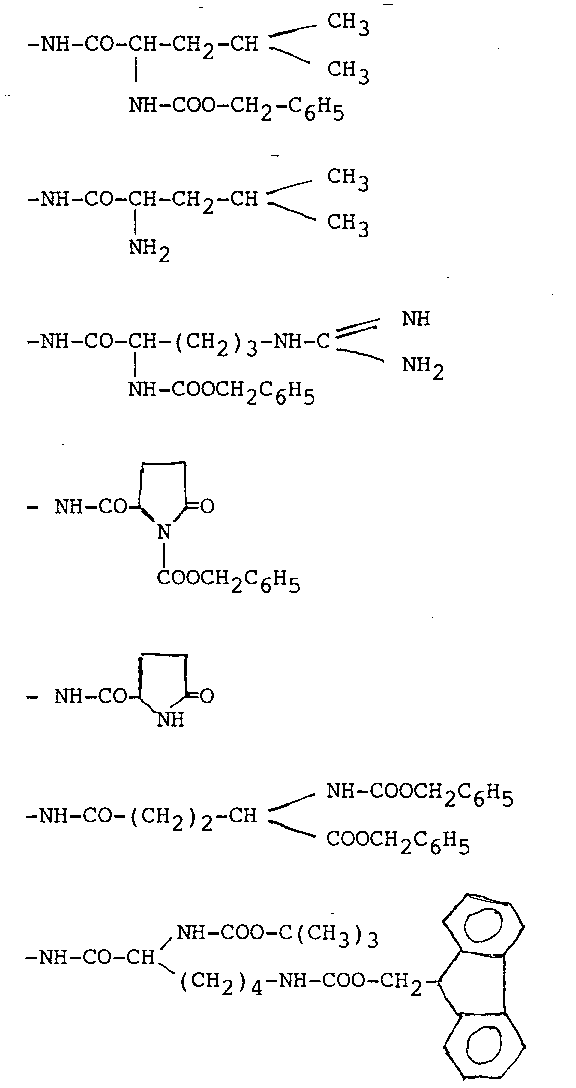

5. Derivat nach Anspruch 2, dadurch gekennzeichnet, daß R⁴ für NHR¹¹, NR⁸R¹¹, SR¹¹ oder OR¹¹ steht.

6. Derivat nach Anspruch 5, dadurch gekennzeichnet, daß R⁴ für NHR¹¹ steht, wobei R¹¹ ein Rest ist, der gewählt ist unter den Resten der Formel:

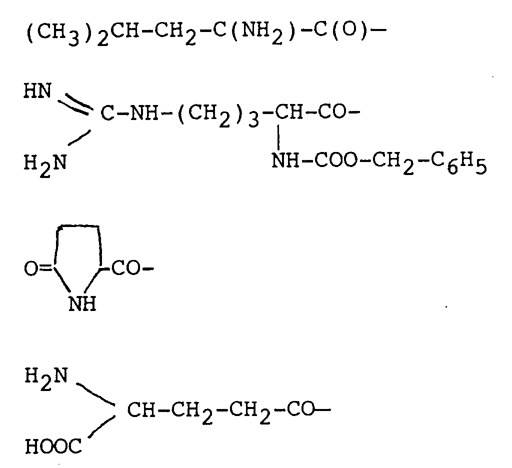

7. Derivat nach Anspruch 6, dadurch gekennzeichnet, daß R¹ für (OCH₂CH₂)₂OCH₃ steht und daß R², R³, R⁵ und R⁶ ein Wasserstoffatom darstellen.

8. Derivat nach Anspruch 4, dadurch gekennzeichnet, daß R¹ für (OCH₂CH₂)₂OCH₃ steht und daß R², R³, R⁵ und R⁶ ein Wasserstoffatom darstellen.

9. Cumarinderivat nach einem der Ansprüche 1 bis 3 und 5, dadurch gekennzeichnet, daß R⁶ ein Wasserstoffatom darstellt.

10. Cumarinderivat nach einem der Ansprüche 2, 3 und 5, dadurch gekennzeichnet, daß R¹ für (OCH₂CH₂)ₘOCH₃ steht.

11. Cumarinderivat nach Anspruch 10, dadurch gekennzeichnet, daß m gleich 2 ist.

12. Cumarinderivat nach Anspruch 1, dadurch gekennzeichnet, daß mindestens einer der Reste R², R³, R⁴ und R⁵ für NH₂, NHR⁸, NR⁸R¹⁰, OH, OR⁸, SH, SR⁸ oder einen substituierten oder nicht substituierten Alkylrest steht.

13. Cumarinderivat nach Anspruch 1, dadurch gekennzeichnet, daß entweder R², R³ und R⁴ oder R³, R⁴ und R⁵ mit dem Benzolkern, an den sie gebunden sind, einen polycyclischen Kern bilden, der drei kondensierte Ringe und ein Stickstoffatom enthält.

14. β-Ketosäureester entsprechend der Formel: 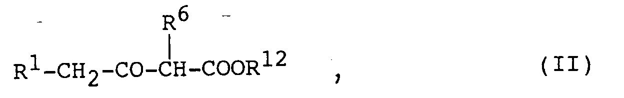 worin R¹ und R⁶ die in Anspruch 1 gegebene Bedeutung haben und R¹² ein Alkyl-, Aryl- oder Cycloalkylrest ist.

15. β-Ketosäureester nach Anspruch 14, dadurch gekennzeichnet, daß R⁶ ein Wasserstoffatom ist.

16. β-Ketosäureester nach einem der Ansprüche 14 und 15, dadurch gekennzeichnet, daß R¹² ein Alkylrest mit 1 bis 4 Kohlenstoffatomen ist.

17. β-Ketosäureester nach Anspruch 16, dadurch gekennzeichnet, daß R¹² der Ethylrest ist.

18. β-Ketosäureester nach einem der Ansprüche 14 bis 17, dadurch gekennzeichnet, daß R¹ den Rest der Formel -
(OCH₂CH₂)ₘOCH₂R⁷
darstellt, worin R⁷ ein Wasserstoffatom oder COOR⁹ ist, wobei R⁹ der Benzylrest ist.

19. Verfahren zur Herstellung eines β-Ketosäureesters der Formel (II) nach einem der Ansprüche 14 bis 18, dadurch gekennzeichnet, daß es die folgenden Schritte umfaßt:
1°) Umwandlung einer Säure der Formel R¹-CH₂-COOH, worin R¹ die in Anspruch 1 gegebene Bedeutung hat, in das entsprechende Imidazolid oder Säurechlorid,
2°) Umsetzung des Imidazolids oder des Säurechlorids mit einem cyclischen Ester der Formel: 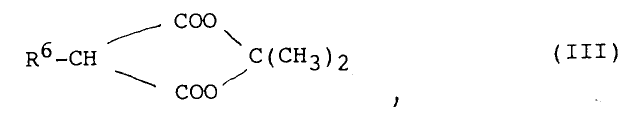 um ein acyliertes Derivat zu bilden, und
3°) Umsetzung des acylierten Derivats mit einem Alkohol der Formel R¹²OH, um den β-Ketosäureester der Formel (II) zu erhalten.

20. Verfahren zur Herstellung eines Cumarinderivats der Formel (I) nach einem der Ansprüche 1 bis 13, dadurch gekennzeichnet, daß es darin besteht, einen β-Ketosäureester der Formel: 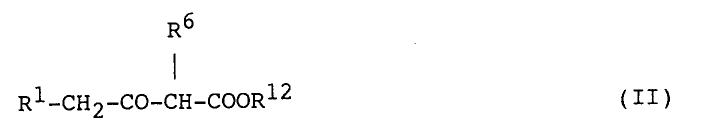 mit einem Phenol der Formel: 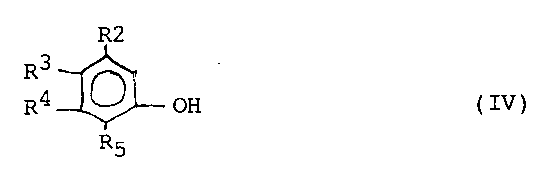 reagieren zu lassen, worin R¹, R², R³, R⁴, R⁵ und R⁶ die oben in Anspruch 1 gegebene Bedeutung haben und R¹² einen Alkyl-, Aryl- oder Cycloalkylrest darstellt.

21. Verwendung der Cumarinderivate nach einem der Ansprüche 3, 4 und 8 zur Synthese eines Enzymsubstrats, das zur Bestimmung dieses Enzyms bestimmt ist.

22. Verwendung der Cumarinderivate nach einem der Ansprüche 5, 6 und 7 als Enzymsubstrat in einem Verfahren zur Bestimmung dieses Enzyms.

## Patentansprüche (Patentansprüche für folgende(n) Vertragsstaat(en): ES)

1. Verfahren zur Herstellung eines Cumarinderivats entsprechend der Formel: 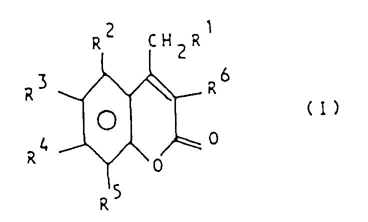 worin
1) R¹ einen Rest darstellt, der den Formeln
- (OCH₂CH₂)ₘOCH₂R⁷ oder
- (OCH(CH₃)CH₂)ₘOCH₂R⁷ entspricht,
worin m eine ganze Zahl von 1 bis 30 ist und R⁷
- ein Wasserstoffatom,
- ein Halogenatom,
- OH,
- OR⁸,
- SH,
- SR⁸,
- COOH,
- COOR⁹.
- CONHR⁸, CONR⁸R¹⁰, CONHNH₂, 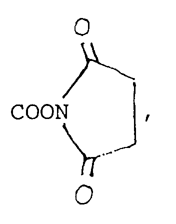
- NH₂,
- NHR⁸,
- NR⁸R¹⁰,
- SO₃H,
- SO₃R⁹ oder
- einen unsubstituierten oder mit mindestens einem Substituenten, der gewählt ist unter den Halogenatomen, dem Phenylrest, CF₃, OH, OR⁸, SH, SR⁸, COOH, COOR⁹, NH₂, NHR⁸, NR⁸R¹⁰, SO₃H und SO₃R⁹, substituierten Alkyl-, Alkoxy- oder Arylrest darstellt,
wo R⁸ ein Alkyl- oder Arylrest ist, R⁹ ein Alkyl- oder Arylrest, NH₄ oder M_{1/v} ist, wobei M ein Metall der Wertigkeit v ist, und R¹⁰ ein Alkyl- oder Arylrest ist oder wo R⁸ und R¹⁰ zusammen einen gesättigten oder ungesättigten Kohlenwasserstoffring bilden können, der gegebenenfalls ein unter O, S und N ausgewähltes Heteroatom enthalten kann,
2) R², R³, R⁴ und R⁵, die gleich oder verschieden sein können,
- ein Wasserstoffatom,
- ein Halogenatom,
- NH₂,
- NHR⁸,
- NR⁸R¹⁰,
- NHR¹¹,
- NR⁸R¹¹,
- SH,
- SR⁸,
- SR¹¹,
- OH,
- OR⁸,
- OR¹¹,
wo R⁸ und R¹⁰ die oben in 1) gegebene Bedeutung haben und R¹¹ einen Acylrest darstellt, der abgeleitet ist von einer Verbindung, die unter den geschützten oder nicht geschützten Aminosäuren, den geschützten oder nicht geschützten Peptiden, den Carbonsäuren und den Fettsäuren, dem Rest PO₃R₂, wobei R ein Wasserstoffatom oder ein Alkylrest ist, oder einem von einem Zucker abgeleiteten Rest gewählt ist, oder
- einen nicht substituierten oder mit mindestens einem Substituenten, der gewählt ist unter den Halogenatomen, OH, OR⁸, SH, SR⁸, COOH, COOR⁹, NH₂, NHR⁸, NR⁸R¹⁰, SO₃H, SO₃R⁹, SR¹¹, OR¹¹, CF₃, NHR¹¹ und NR⁸R¹¹, substituierten Alkyl- oder Alkoxyrest darstellen, wobei R⁸, R⁹, R¹⁰ und R¹¹ die oben in 1) gegebene Bedeutung haben, oder
3) R² und R³, R³ und R⁴ oder R⁴ und R⁵ zusammen einen ringförmigen, gesättigten oder ungesättigten Kern von 5 bis 8 Kohlenstoffatomen oder einen heterocyclischen Kern bilden, der mindestens ein unter N, O und S gewähltes Heteroatom enthält, oder
4) R³, R⁴ und R⁵ mit dem Benzolkern, an den sie gebunden sind, einen polycyclischen Kern mit drei kondensierten Ringen bilden, der gegebenenfalls ein oder mehrere unter N, O und S gewählte Heteroatome enthält, oder
5) R², R³ und R⁴ mit dem Benzolkern, an den sie gebunden sind, einen polycyclischen Kern mit drei kondensierten Ringen bilden, der gegebenenfalls ein oder mehrere unter N, O und S gewählte Heteroatome enthält, und
6) R⁶
- ein Wasserstoffatom,
- ein Halogenatom,
- COOH,
- COOR⁹, wobei R⁹ die oben gegebene Bedeutung hat, oder
- einen nicht substituierten oder mit mindestens einem Substituenten, der gewählt ist unter den Halogenatomen, OH, SH, COOH, COOR⁹, OR⁸, SR⁸, SO₃H und SO₃R⁹, substituierten Alkylrest darstellt, wobei R⁸ und R⁹ die oben in 1) gegebene Bedeutung haben,
dadurch gekennzeichnet, daß es darin besteht, einen β-Ketosäureester der Formel: 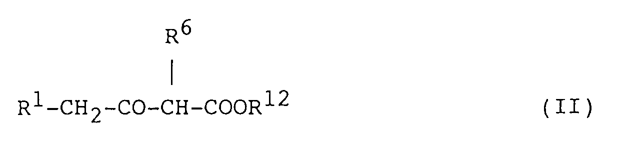 mit einem Phenol der Formel: 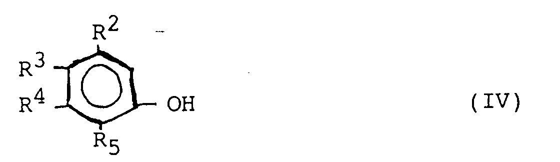 reagieren zu lassen, worin R¹, R², R³, R⁴, R⁵ und R⁶ die oben in Abschnitt 1) bis 6) gegebene Bedeutung haben und R¹² einen Alkyl-, Aryl- oder Cycloalkylrest darstellt.

2. Verfahren nach Anspruch 1, dadurch gekennzeichnet, daß mindestens einer der Substituenten R², R³, R⁴ und R⁵ für NH₂, NHR¹¹, NHR⁸, NR⁸R¹¹, SH, OH, SR¹¹ oder OR¹¹ steht, wobei R⁸ und R¹¹ die in Anspruch 1 gegebene Bedeutung haben.

3. Verfahren nach Anspruch 2, dadurch gekennzeichnet, daß R⁴ für NH₂, NHR⁸, NHR¹¹, SH oder OH steht und daß R², R³ und R⁵ ein Wasserstoffatom darstellen.

4. Verfahren nach Anspruch 3, dadurch gekennzeichnet, daß R⁴ für NH₂, OH oder einen Rest steht, der gewählt ist unter den Resten der Formel:

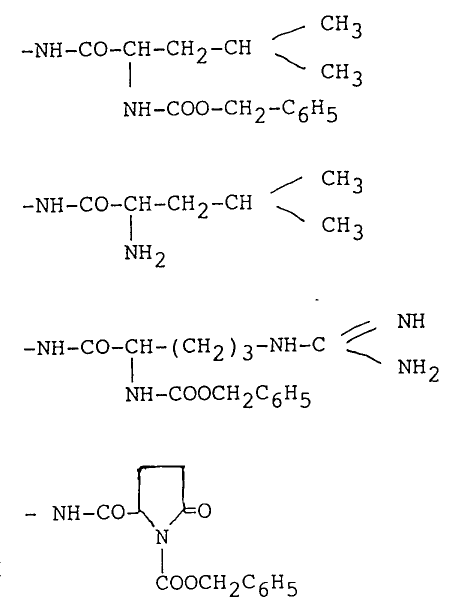

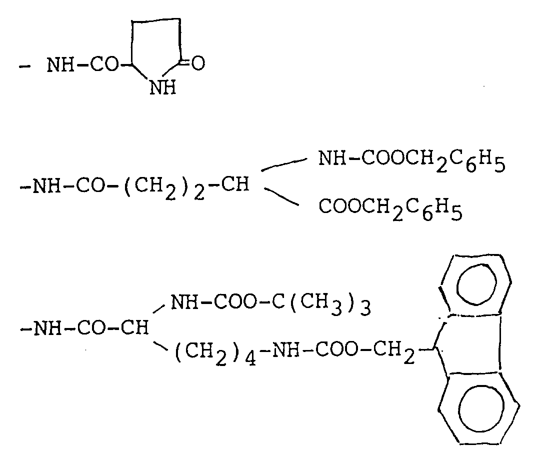

5. Verfahren nach Anspruch 2, dadurch gekennzeichnet, daß R⁴ für NHR¹¹, NR⁸R¹¹, SR¹¹ oder OR¹¹ steht.

6. Verfahren nach Anspruch 5, dadurch gekennzeichnet, daß R⁴ für NHR¹¹ steht, wobei R¹¹ ein Rest ist, der gewählt ist unter den Resten der Formel:

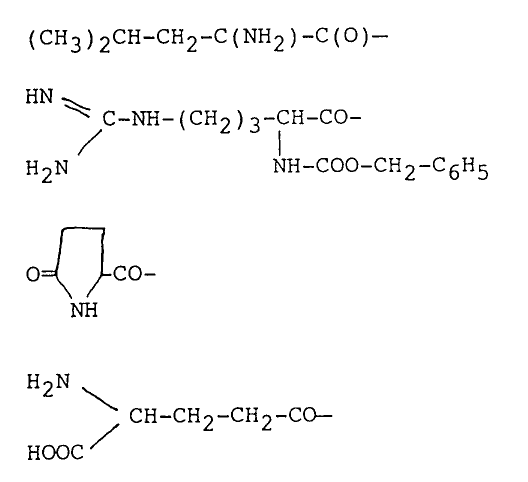

7. Verfahren nach Anspruch 6, dadurch gekennzeichnet, daß R¹ für (OCH₂CH₂)₂OCH₃ steht und daß R², R³, R⁵ und R⁶ ein Wasserstoffatom darstellen.

8. Verfahren nach Anspruch 4, dadurch gekennzeichnet, daß R¹ für (OCH₂CH₂)₂OCH₃ steht und daß R², R³, R⁵ und R⁶ ein Wasserstoffatom darstellen.

9. Verfahren nach einem der Ansprüche 1 bis 3 und 5, dadurch gekennzeichnet, daß R⁶ ein Wasserstoffatom darstellt.

10. Verfahren nach einem der Ansprüche 2, 3 und 5, dadurch gekennzeichnet, daß R¹ für (OCH₂CH₂)ₘOCH₃ steht.

11. Verfahren nach Anspruch 10, dadurch gekennzeichnet, daß m gleich 2 ist.

12. Verfahren nach Anspruch 1, dadurch gekennzeichnet, daß mindestens einer der Reste R², R³, R⁴ und R⁵ für NH₂, NHR⁸, NR⁸R¹⁰, OH, OR⁸, SH, SR⁸ oder einen substituierten oder nicht substituierten Alkylrest steht.

13. Verfahren nach Anspruch 1, dadurch gekennzeichnet, daß entweder R², R³ und R⁴ oder R³, R⁴ und R⁵ mit dem Benzolkern, an den sie gebunden sind, einen polycyclischen Kern bilden, der drei kondensierte Ringe und ein Stickstoffatom enthält.

14. Verfahren zur Herstellung eines β-Ketosäureesters entsprechend der Formel 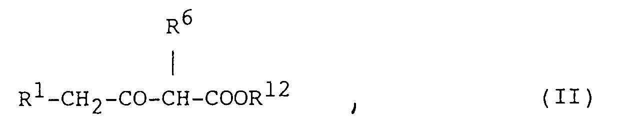 worin R¹ und R⁶ die in Anspruch 1 gegebene Bedeutung haben und R¹² ein Alkyl-, Aryl- oder Cycloalkylrest ist, dadurch gekennzeichnet, daß es die folgenden Schritte umfaßt:
1°) Umwandlung einer Säure der Formel R¹-CH₂-COOH, worin R¹ die in Anspruch 1 gegebene Bedeutung hat, in das ensprechende Imidazolid oder Säurechlorid,
2°) Umsetzung des Imidazolids oder des Säurechlorids mit einem cyclischen Ester der Formel: 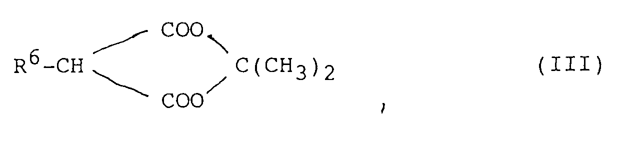 um ein acyliertes Derivat zu bilden, und
3°) Umsetzung des acylierten Derivats mit einem Alkohol der Formel R¹²OH, um den β-Ketosäureester der Formel (II) zu erhalten.

15. Verfahren nach Anspruch 14, dadurch gekennzeichnet, daß R⁶ ein Wasserstoffatom ist.

16. Verfahren nach einem der Ansprüche 14 und 15, dadurch gekennzeichnet, daß R¹² ein Alkylrest mit 1 bis 4 Kohlenstoffatomen ist.

17. Verfahren nach Anspruch 16, dadurch gekennzeichnet, daß R¹² der Ethylrest ist.

18. Verfahren nach einem der Ansprüche 14 bis 17, dadurch gekennzeichnet, daß R¹ den Rest der Formel
- (OCH₂CH₂)ₘOCH₂R⁷
darstellt, worin R⁷ ein Wasserstoffatom oder COOR⁹ ist, wobei R⁹ der Benzylrest ist.

19. Verwendung der durch das Verfahren nach einem der Ansprüche 3, 4 und 8 erhaltenen Cumarinderivate zur Synthese eines Enzymsubstrats, das zur Bestimmung dieses Enzyms bestimmt ist.

20. Verwendung der durch das Verfahren nach einem der Ansprüche 5, 6 und 7 erhaltenen Cumarinderivate als Enzymsubstrat in einem Verfahren zur Bestimmung dieses Enzyms.
